# EUROPEAN PATENT SPECIFICATION

(11) **EP 1 725 552 B1**
(45) Date of publication and mention of the grant of the patent: **25.06.2008**
(21) Application number: 05729711.1
(22) Date of filing: 10.03.2005
(51) Int. Cl.: C07D 453/02, A61K 31/439, A61P 11/00, A61P 13/00

(54) **NEW QUATERNIZED QUINUCLIDINE ESTERS**
NEUE QUATERNISIERTE CHINUCLIDINESTER
NOUVEAUX ESTERS DE QUINUCLIDINE QUATERNISES

(30) Priority: 15.03.2004 ES 200400638
(43) Date of publication of application: 29.11.2006
(73) Proprietor: Laboratorios Almirall, S.A., 08022 Barcelona (ES)
(72) Inventor: FERNANDEZ FORNER, Maria Dolors, E-08025 Barcelona (ES); PRAT QUINONES, Maria, E-08003 Barcelona (ES); BUIL ALBERO, Maria Antonia, E-08029 Barcelona (ES)
(74) Representative: Winkler, Andreas Fritz Ernst
(86) International application number: PCT/EP2005/002523
(87) International publication number: WO 2005/090342

(56) References cited:
- WO-A-01/04118
- WO-A-02/053564
- WO-A-20/04005285
- WO-A-20/04096800

## Description

This invention relates to new therapeutically useful quinuclidine derivatives, to some processes for their preparation and to pharmaceutical compositions containing them.

The novel structures according to the invention are antimuscarinic agents with a potent and long lasting effect. In particular, these compounds show high affinity for muscarinic M3 receptors. This subtype of muscarinic receptor is present in glands and smooth muscle and mediates the excitatory effects of the parasympathetic system on glandular secretion and on the contraction of visceral smooth muscle (Chapter 6, Cholinergic Transmission, in H.P. Rang et al., Pharmacology, Churchill Livingstone, New York, 1995).

M3 antagonists are therefore known to be useful for treating diseases characterised by an increased parasympathetic tone, by excessive glandular secretion or by smooth muscle contraction (R.M. *Eglen and S.S. Hegde, (1997), Drug News Perspect., 10(8):462-469).

Examples of this kind of diseases are respiratory disorders such as chronic obstructive pulmonary disease (COPD), bronchitis, bronchial hyperreactivity, asthma, cough and rhinitis; urological disorders such as urinary incontinence, pollakiuria, neurogenic or unstable bladder, cystospasm and chronic cystitis; gastrointestinal disorders such as irritable bowel syndrome, spastic colitis, diverticulitis and peptic ulceration; and cardiovascular disorders such as vagally induced sinus bradycardia (Chapter 7, Muscarinic Receptor Agonists and Antagonists, in Goodman and Gilman's The Pharmacological Basis of Therapeutics, 10the edition, McGraw Hill, New York, 2001).

The compounds of the invention can be used alone or in association with other drugs commonly regarded as effective in the treatment of these diseases. For example, they can be administered in combination with β₂-agonists, steroids, antiallergic drugs, phosphodiesterase IV inhibitors and/or leukotriene D4 (LTD4) antagonists for simultaneous, separate or sequential use in the treatment of a respiratory disease. The claimed compounds are useful for the treatment of the respiratory diseases detailed above in association with β₂-agonists, steroids, antiallergic drugs or phosphodiesterase IV inhibitors.

Compounds with related structures have been described as anti-cholinergic and/or anti-spasmodics agents in several patents.

For example, FR 2012964 describes quinuclidinol derivatives of the formula 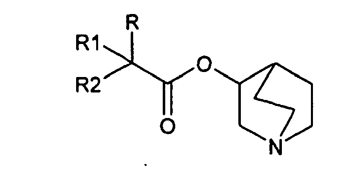 in which R is H, OH or an alkyl group having 1 to 4 carbon atoms; R¹ is a phenyl or thienyl group; and R² is a cyclohexyl, cyclopentyl or thienyl group, or, when R is H, R¹ and R² together with the carbon atom to which they are attached, form a tricyclic group of the formula: 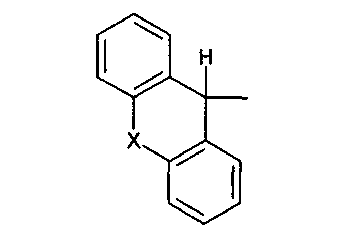 in which X is -O-, -S- or -CH₂-,
or an acid addition or quaternary ammonium salt thereof.

In United States Patent US 4,465,834 a class of anticholinergic drugs having the formula 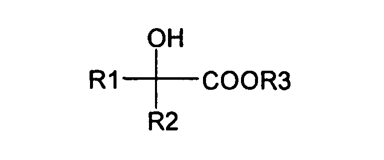 are described, in which R¹ is a carbocyclic or branched aliphatic group of 3 to 8 carbon atoms (such as phenyl, cyclohexyl, cyclopentyl, cyclopropyl, cycloheptyl, and isopropyl), R² is a branched or linear aliphatic group containing 3 to 10 carbon atoms with 1 or 2 olefinic or acetylenic bonds, or is a phenylethinyl, a styryl, or an ethynyl group, and R³ is an alkyl or cyclic group of 4 to 12 carbon atoms containing a tertiary amino nitrogen. The compounds of the invention are also claimed as either the free base or the acid-addition and quaternary ammonium salt forms thereof.

In United States Patent 4,843,074 products of formula 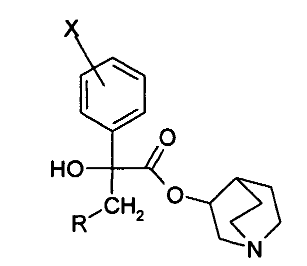 are described, wherein X = H, halogen, lower alkyl, lower alkoxy, hydroxy and R = morpholinyl, thiomorpholinyl, piperidinyl, 1,4-dioxa-8-azaspiro[4,5]decanyl, 4-(2,6-dimetylmorpholinyl), 4-ketopiperidinyl, 4-hydroxypiperidinyl, 4-substituted piperazinyl. The lower alkyl halide quaternary salts and pharmaceutically acceptable acid addition salts are included in the invention.

US Patent 4,644,003 describes esters of 3-quinuclidinol of alpha disubstituted glycolic acids 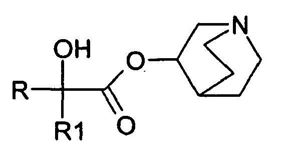 and their pharmaceutically acceptable salts,
where R is phenyl, unsubstituted or substituted with up to three substituents including alkoxy, halogen, nitro, amino, alkylamino, dialkylamino, acylamino, and trifluoromethyl; and wherein R¹ is hydrogen, alkyl, cycloalkyl, alkenyl, cycloalkenyl, alkynyl, alkyloxyalkyl, cycloalkyloxyalkyl, haloalkyl or haloalkenyl.

In WO 92/04346 are described compounds of formula 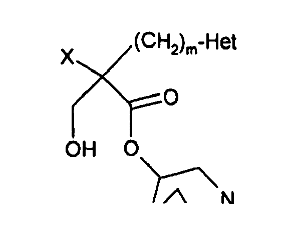 and their pharmaceutically acceptable salts, where X is a phenyl (optionally substituted) or a thienyl group and "Het" is either (a) a five membered nitrogen-containing heterocyclic group, (b) an oxadiazolyl or thiadiazolyl group, or (c) a six membered nitrogen-containing heterocyclic group, and m is 1 or 2. (For a more detailed description, see the above mentioned publication)

Azoniabicyclic compounds of a general structure related to the compounds of the invention are disclosed in WO 01/04118 and WO 02/053564.

WO 2004/096800 is directed to esters of quaternized 3-quinuclidinols of formula 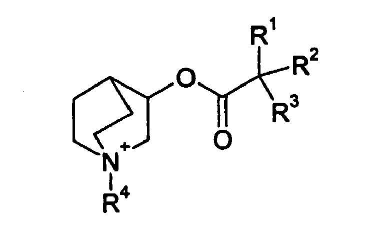 wherein the quaternising group (R⁴) is a C₁₋₈ alkyl group substituted, among others, by -OR¹¹, -OCO-R¹³ or -COO-R¹⁴ wherein R¹¹ is hydrogen, C₁₋₈-alkyl, C₁₋₈-alkyl-C₁₋₈-alkoxy or C₁₋₈-alkyl-O-C₃₋₁₅ carbocyclic group, R¹³ is C₁₋₈-alkyl or a C₃₋₁₅-carbocyclic group and R¹⁴ is hydrogen, a C₃₋₁₅-carbocyclic group, C₁₋₈-alkenyl, or C₁₋₈-alkyl optionally substituted by a C₃₋₁₅-carbocyclic group and discloses specifically, among others, the compounds: 1-Allyloxycarbonylmethyl-3-(2-hydroxy-2,2-di-thiophen-2-yl-acetoxy)-1-azonia-bicyclo-[2.2.2]octane and 1-carboxymethyl-3-(2-hydroxy-2,2-di-thiophen-2-yl-acetoxy)-1-azonia-bicyclo-[2.2.2]octane.

The present invention provides new quinuclidine ester derivatives with potent antagonist activity at muscarinic M3 receptors which have the chemical structure described in formula (I): 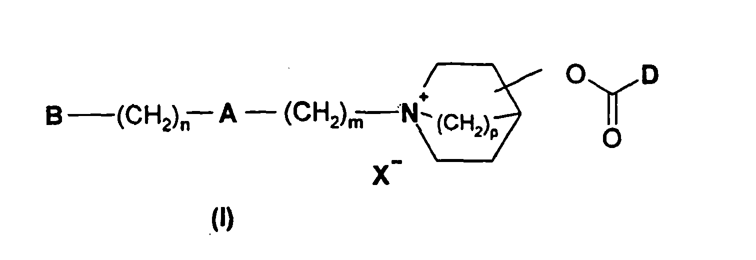 B represents a hydrogen atom or a group selected from -R¹, -OR¹, hydroxy, -O(CO)R¹, cyano and an optionally substituted non-aromatic heterocyclyl containing one or more heteroatoms, wherein
R¹ is selected from the group consisting of hydrogen atoms, optionally substituted C₁₋₈ alkyl, optionally substituted C₂₋₈ alkenyl and optionally substituted C₃- C₈ cycloalkyl n is an integer from 0 to 4;
A represents a group selected from -CH₂-, -CH=CR³-, -CR³=CH-, -CR³R⁴-, -O-, -CO-, -O-(CH₂)₂-O- wherein R³ and R⁴ each independently represent a hydrogen atom or a C₁₋₈ alkyl group;
m is an integer from 0 to 8;
p is an integer from 1 to 2
and the substitution in the azoniabicyclic ring may be in the 2, 3 or 4 position including all possible configurations of the asymmetric centers;
D is a group selected from: 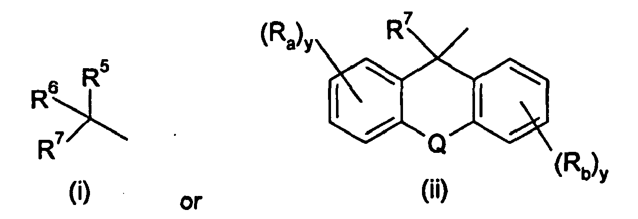 wherein R⁵ represents a group selected from phenyl, 2-thienyl, 3-thienyl, 2-furanyl, 3-furanyl which group may be optionally substituted by one or more substitutent Rₐ;
R⁶ represents a group selected from 2-thienyl, 3-thienyl, 2-furanyl, 3-furanyl, C₃-C₈ cycloalkyl, C₃-C₈ cycloalkenyl, C₁-C₈ alkyl, C₂-C₈ alkenyl, C₂-C₈ alkynyl, benzyl and phenylethyl which group may be optionally substituted by one or more substitutents R_{b};
R⁷ represents a hydrogen atom or a group selected from hydroxyl, hydroxymethyl and methyl;
Q represents a single bond or a group selected from -CH₂-, -CH₂CH₂-, -O-, -O-CH₂-, -S-, -S-CH₂-, and -CH=CH-;
Rₐ and R_{b} independently represent a group selected from halogen atoms, optionally substituted C₁-C₈ alkyl, optionally substituted C₁-C₈ alkoxy, hydroxy, trifluoromethyl, nitro, cyano, -COOR⁸, -NR⁸R⁹ wherein R⁸ and R⁹ independently represent a hydrogen atom or a C₁-C₈ alkyl group.
y is an integer from 0 to 3
X⁻ represents a pharmaceutically acceptable anion of a mono or polyvalent acid;
provided that the group B-(CH₂)ₙ-A-(CH₂)ₘ- is not a linear C₁₋₄ alkyl group and further provided that the compound is not one of:
1-Allyloxycarbonylmethyl-3-(2-hydroxy-2,2-di-thiophen-2-yl-acetoxy)-1-azonia-bicyclo-[2.2.2]octane; and
1-carboxymethyl-3-(2-hydroxy-2,2-di-thiophen-2-yl-acetoxy)-1-azonia-bicyclo-[2.2.2]octane.

Other aspects of the present invention are: a) a process for the preparation of the compounds of formula (I), b) pharmaceutical compositions comprising an effective amount of said compounds, and c) the use of said compounds in the manufacture of a medicament for the treatment of respiratory, urinary and/or gastrointestinal diseases.

As used herein the term lower alkyl embraces optionally substituted, linear or branched radicals having from 1 to 8, preferably 1 to 6 and more preferably 1 to 4 carbon atoms.

Examples include methyl, ethyl, n-propyl, i-propyl, n-butyl, sec-butyl and tert-butyl, n-pentyl, 1-methylbutyl, 2-methylbutyl, isopentyl, 1-ethylpropyl, 1,1-dimethylpropyl, 1,2-dimethylpropyl, n-hexyl or 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl, trifluoromethyl and iso-hexyl radicals.

As used herein, the term lower alkenyl embraces optionally substituted, linear or branched, mono or polyunsaturated radicals having 2 to 8, preferably 2 to 6 and more preferably 2 to 4 carbon atoms. In particular it is preferred that the alkenyl radicals are mono or diunsaturated.

Examples include vinyl, allyl, 1-propenyl, isopropenyl, 1-butenyl, 2-butenyl, 3-butenyl, 1-pentenyl, 2-pentenyl, 3-pentenyl and 4-pentenyl radicals.

As used herein, the term lower alkynyl embraces optionally substituted, linear or branched, mono or polyunsaturated radicals having 2 to 8, preferably 2 to 6 and more preferably 2 to 4 carbon atoms. In particular, it is preferred that the alkynyl radicals are mono or diunsaturated.

Examples include 1-propynyl, 2-propynyl, 1-butynyl, 2-butynyl and 3-butynyl radicals.

As used herein, the term lower alkoxy embraces optionally substituted, linear or branched oxy-containing radicals each having alkyl portions of 1 to 8 carbon atoms, preferably 1 to 6 and more preferably 1 to 4 carbon atoms.

Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy or 2-hydroxypropoxy.

As used herein, the term cycloalkyl embraces saturated carbocyclic radicals and, unless otherwise specified, a cycloalkyl radical typically has from 3 to 7 carbon atoms.

A cycloalkyl radical is typically unsubstituted or substituted with 1, 2 or 3 substituents which may be the same or different. When a cycloalkyl radical carries 2 or more substituents, the substituents may be the same or different.

Examples include cyclopropyl, cyclobutyl, cyclopentyl, cyclohexyl and cycloheptyl. It is preferably cyclopropyl, cyclopentyl or cyclohexyl.

As used herein, the term cycloalkenyl embraces partially unsaturated carbocyclic radicals and, unless otherwise specified, a cycloalkenyl radical typically has from 3 to 7 carbon atoms.

A cycloalkenyl radical is typically unsubstituted or substituted with 1, 2 or 3 substituents which may be the same or different. When a cycloalkenyl radical carries 2 or more substituents, the substituents may be the same or different.

Examples include cyclobutenyl, cyclopentenyl, cyclohexenyl and cycloheptenyl. It is preferably cyclopentenyl or cyclohexenyl.

As used herein, the term heterocyclyl radical embraces typically a non-aromatic, saturated or unsaturated C₃-C₁₀ carbocyclic ring, such as a 5, 6 or 7 membered radical, in which one or more, for example 1, 2, 3 or 4 of the carbon atoms preferably 1 or 2 of the carbon atoms are replaced by a heteroatom selected from N, O and S excluding the group benzo[1,3]dioxolyl. Saturated heterocyclyl radicals are preferred. A heterocyclic radical may be a single ring or two or more fused rings wherein at least one ring contains a heteroatom. When a heterocyclyl radical carries 2 or more substituents, the substituents may be the same or different.

An heterocyclyl radical is typically unsubstituted or substituted with 1, 2 or 3 substituents which may be the same or different. The substituents are preferably selected from halogen atoms, preferably fluorine atoms, hydroxy groups, oxo groups, alkyl groups having from 1 to 4 carbon atoms and alkoxy groups having from 1 to 4 carbon atoms. When an heterocyclyl radical carries 2 or more substituents, the substituents may be the same or different.

Examples of non-aromatic heterocyclic radicals include piperidyl, pyrrolidinyl, pyrrolinyl, piperazinyl, morpholinyl, thiomorpholinyl, pyrazolinyl, pirazolidinyl, quinuclidinyl, imidazolidinyl, oxiranyl and azaridinyl.

As used herein, the term halogen atom embraces chlorine, fluorine, bromine or iodine atom typically a fluorine, chlorine or bromine atom, most preferably chlorine or fluorine.

As used herein when a group or radical is said to be optionally substituted it is meant that it may have some of its hydrogen atoms replaced by up to 3 substituents selected from the group comprising halogen atoms, hydroxy groups, alkyl groups having from 1 to 4 carbon atoms and alkoxy groups having from 1 to 4 carbon atoms. When the group carries more than one substituent it is preferred that the substituents are bound to different atoms in the group.

As used herein, the term mono or polyvalent acid embraces pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, formic, acetic, trifluoroacetic, methanesulphonic, ethanesulphonic, benzenesulphonic or p-toluenesulphonic acid.

Wherein the compounds of the present invention contain one or more chiral centers all configurations of the chiral center are covered and in particular enantiomers or diastereomers arising from the multiple configurations are within the scope of the present invention. Non limiting examples of the chiral centers which may be present in the compounds of the present invention are the quaternary nitrogen atom of the azoniabicyclic ring, the carbon atom in the azoniabicyclic ring to which the group D-COO- is attached and the carbon atom by which group D is linked to the ester group.

In a preferred embodiment of the present invention preferred compounds of formula (I) are those wherein B represents a hydrogen atom or a group selected from -R¹, -OR¹, hydroxy, -O(CO)R¹, cyano and an optionally substituted non-aromatic heterocyclyl containing one or more heteroatoms, wherein
R¹ is selected from the group consisting of hydrogen atoms, optionally substituted C₁₋₈ alkyl, optionally substituted C₂₋₈ alkenyl and optionally substituted C₃- C₈ cycloalkyl n is an integer from 0 to 4;
A represents a group selected from -CH₂-, -CH=CR³-, -CR³=CH-, -CR³R⁴-, -O-, -CO-, -O-(CH₂)₂-O- wherein R³ and R⁴ each independently represent a hydrogen atom or a C₁₋₈ alkyl group;
m is an integer from 0 to 8;
p is an integer from 1 to 2
and the substitution in the azoniabicyclic ring may be in the 2, 3 or 4 position including all possible configurations of the asymmetric centers;
D is a group selected from: 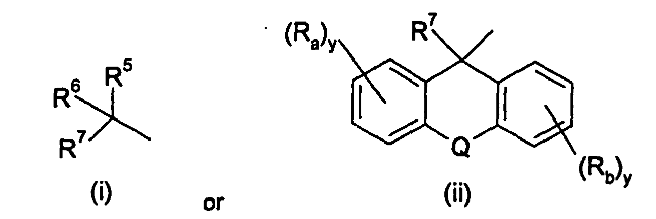 wherein R⁵ represents a group selected from phenyl, 2-thienyl, 3-thienyl, 2-furanyl, 3-furanyl which group may be optionally substituted by one or more substitutent Rₐ;
R⁶ represents a group selected from 2-thienyl, 3-thienyl, 2-furanyl, 3-furanyl, C₃-C₈ cycloalkyl, C₃-C₈ cycloalkenyl, C₁-C₈ alkyl, C₂-C₆ alkenyl, C₂-C₈ alkynyl, benzyl and phenylethyl which group may be optionally substituted by one or more substitutents R_{b};
R⁷ represents a hydrogen atom or a group selected from hydroxyl, hydroxymethyl and methyl;
Q represents a single bond or a group selected from -CH₂-, -CH₂CH₂-, -0-, -O-CH₂-, -S-, -S-CH₂-, and -CH=CH-;
Rₐ and R_{b} independently represent a group selected from halogen atoms, optionally-substituted C₁-C₈ alkyl, optionally substituted C₁-C₈ alkoxy, hydroxy, trifluoromethyl, nitro, cyano, -COOR⁸, -NR⁸R⁹ wherein R⁸ and R⁹ independently represent a hydrogen atom or a C₁-C₈ alkyl group.
y is an integer from 0 to 3
X⁻ represents a pharmaceutically acceptable anion of a mono or polyvalent acid;
provided that the group B-(CH₂)ₙ-A-(CH₂)ₘ- is neither a linear C₁₋₄ alkyl group nor a C₁₋₈ alkyl group substituted by -OR¹¹, -OCO-R¹³ or -COO-R¹⁴ wherein R¹¹ is hydrogen, C₁₋₈-alkyl, C₁₋₈-alkyl-C₁₋₈-alkoxy or C₁₋₈-alkyl-O-C₃₋₁₅ carbocyclic group, R¹³ is C₁₋₈-alkyl or a C₃₋₁₅-carbocyclic group and R¹⁴ is hydrogen, a C₃₋₁₅-carbocyclic group, C₁₋₈-alkenyl, or C₁₋₈-alkyl optionally substituted by a C₃₋₁₅-carbocyclic group

In an embodiment of the present invention preferred compounds of formula (I) are those wherein A represents a group selected from -CH₂-, -CH=CR³-, -CR³=CH-, -CR³R⁴-, -O-, -O-(CH₂)₂-O- wherein R³ and R⁴ each independently represent a hydrogen atom or a C₁₋₈ alkyl group;

In another embodiment of the present invention preferred compounds of formula (I) are those wherein B is selected from the group consisting of hydrogen atoms, hydroxy groups, optionally substituted C₁-C₈ alkyl, optionally substituted C₂-C₈ alkenyl, optionally substituted C₃-C₈ cycloalkyl groups and non-aromatic heterocyclyl groups substituted at least with a hydroxy group.

In still another embodiment of the present invention the azoniabicyclo group is substituted on the nitrogen atom with a group selected from allyl, 4-methylpent-3-enyl, isopropyl, cyclopropylmethyl, isobutyl, heptyl, cyclohexylmethyl, 3-cyclohexylpropyl, 3,7-dimethylocta-(E)-2,6-dienyl, 2-hydroxyethyl, 3-hydroxypropyl, 4-hydroxybutyl, 5-hydroxypentyl, 6-hydroxyhexyl, 2-ethoxyethyl, 2-(2-hydroxyethoxy)ethyl, 2-(2-methoxyethoxy)ethyl, oxiranylmethyl, 2-[1,3]dioxolan-2-ylethyl, 2-[2-(2-hydroxyethoxy)-ethoxy]ethyl, 3-[1,3]dioxolan-2-ylpropyl, 2-ethoxycarbonylethyl, 3-ethoxycarbonylpropyl, 4-ethoxycarbonylbutyl, 4-acetoxybutyl, 2-cyanoethyl, 3-cyanopropyl, 4-cyanobutyl, 6-cyanohexyl, 4,4,4-trifluorobutyl, 3-(4-hydroxypiperidin1-yl)propyl and 4-(4-hydroxypiperidin1-yl)butyl. More preferably the azoniabicyclo group is substituted on the nitrogen atom with a group selected from allyl, 4-methylpent-3-enyl, isopropyl, cyclopropylmethyl, isobutyl, heptyl, 2-hydroxyethyl, 3-hydroxypropyl, 4-hydroxybutyl, 5-hydroxypentyl, 6-hydroxyhexyl, 2-(2-methoxyethoxy)ethyl, 2-(2-hydroxyethoxy)ethyl, 4-ethoxycarbonylbutyl, 4-acetoxybutyl, 3-cyanopropyl and 4-cyanobutyl. It is particularly preferred that the azoniabicyclo group is substituted on the nitrogen atom with a group selected from allyl, 4-methylpent-3-enyl and n-heptyl, more preferably with a group selected from allyl and n-heptyl and most preferably with an allyl group.

In another embodiment of the present invention p is 2.

In still another embodiment of the present invention the substitution in the azoniabicyclic ring is in the 3 position and includes all possible configurations of the asymmetric carbon. More preferably the configuration of carbon 3 in the azoniabicyclic ring is R configuration.

In another embodiment of the present invention R⁵ represents an unsubstituted phenyl, 2-thienyl, 3-thienyl, 2-furyl or 3-furyl group.

In another embodiment of the present invention R⁸ represents a 2-thienyl, 3-thienyl, 2-furyl 3-furyl or cyclopentyl group.

In still another embodiment of the present invention the group of formula -O-CO-C(R⁵)(R⁶)(R⁷) represents a group selected from 2,2-dithien-2-ylacetoxy, 2,2-dithien-2-ylpropionyloxy, 2-hydroxy-2,2-dithien-2-ylacetoxy, 2-hydroxy-2-phenyl-2-thien-2-ylacetoxy, 2-fur-2-yl-2-hydroxy-2-phenylacetoxy, 2-fur-2-yl-2-hydroxy-2-thien-2-ylacetoxy, (2*)-2-hydroxy-2,3-diphenylpropionyloxy, 2-hydroxy-2-thien-2-ylpent-4-enoyloxy, (2S)-2-cyclopentyl-2-hydroxy-2-thien-2-ylacetoxy and (2R)-2-cyclopentyl-2-hydroxy-2-thien-2-ylacetoxy. More preferably the group -O-CO-C(R⁵)(R⁶)(R⁷) represents a group selected from 2-cyclopentyl-2-hydroxy-2-thien-2-ylacetoxy; 2,2-dithien-2-ylacetoxy, 2-hydroxy-2,2-dithien-2-ylacetoxy, 2,2-dithien-2-ylpropionyloxy; 2-hydroxy-2-phenyl-2-thien-2-ylacetoxy, 2-fur-2-yl-2-hydroxy-2-thien-2-ylacetoxy and 2-fur-2-yl-2-hydroxy-2-phenylacetoxy. Still more preferably the group -O-CO-C(R⁵)(R⁶)(R⁷) represents a group selected from 2-hydroxy-2,2-dithien-2-ylacetoxy and (2S)-2-cyclopentyl-2-hydroxy-2-thien-2-ylacetoxy and most preferably a 2-hydroxy-2,2-dithien-2-ylacetoxy group.

In still another embodiment of the present invention the group of formula D-COO- represents a group selected from 9-methyl-9H-fluorene-9-carbonyloxy, 9-hydroxy-9H-fluorene-9-carbonyloxy, 9H-xanthene-9-carbonyloxy, 9-methyl-9H-xanthene-9-carbonyloxy, 9-hydroxy-9H-xanthene-9-carbonyloxy, 9,10-dihydroanthracene-9-carbonyloxy and 10,11-dihydro-5H-dibenzo[a,d]cycloheptene-5-carbonyloxy. More preferably the group of formula D-COO-represents a group selected from 9-methyl-9H-fluorene-9-carbonyloxy, 9-hydroxy-9H-fluorene-9-carbonyloxy, 9H-xanthene-9-carbonyloxy, 9-methyl-9H-xanthene-9-carbonyloxy and 9-hydroxy-9H-xanthene-9-carbonyloxy and most preferably selected from 9-methyl-9H-xanthene-9-carbonyloxy and 9-hydroxy-9H-xanthene-9-carbonyloxy.

In another embodiment of the present invention the carbon substituted by R⁵, R⁶ and R⁷ has R configuration.

In another embodiment of the present invention the carbon substituted by R⁵, R⁶ and R⁷ has S configuration.

The following compounds are intended to illustrate but not to limit the scope of the present invention:
(3R)-1-Allyl-3-(2,2-dithien-2-ylacetoxy)-1-azoniabicyclo[2.2.2]octane bromide
(3R)-3-(2,2-Dithien-2-ylacetoxy)-1-(4-methylpent-3-enyl)-1-azoniabicyclo[2.2.2]octane bromide
(3R)-1-Allyl-3-(2,2-dithien-2-ylpropionyloxy)-1-azoniabicyclo[2.2.2]octane bromide
(3R)-1-(4-Methylpent-3-enyl)-3-(2,2-dithien-2-ylpropionyloxy)-1-azoniabicyclo[2.2.2]octane bromide
(3R)-3-(2-Hydroxy-2,2-dithien-2-ylacetoxy)-1-isopropyl-1-azoniabicyclo[2.2.2]octane trifluoroacetate
(3R)-1-Cyclopropylmethyl-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-isobutyl-1-azoniabicyclo[2.2.2]octane trifluoroacetate
(3R)-1-Heptyl-3-(2-hydroxy-2,2-dithien-2-ylacetoxy)-1-azoniabicyclo[2.2.2]octane bromide
(3R)-1-Cyclohexylmethyl-3-(2-hydroxy-2,2-dithien-2-ylacetoxy)-1-azonabicyclo[2.2.2]octane trifluoroacetate
(3R)-1-(3-Cyclohexylpropyl)-3-(2-hydroxy-2,2-dithien-2-ylacetoxy)-1-azoniabicyclo[2.2.2]octane trifluoroacetate
(3R)-1-Allyl-3-(2-hydroxy-2,2-dithien-2-ylacetoxy)-1-azoniabicyclo[2.2.2]octane bromide
(3R)-3-(2-Hydroxy-2,2-dithien-2-ylacetoxy)-1-(4-methylpent-3-enyl)-1-azoniabicyclo[2.2.2]octane bromide
(3R)-1-(3,7-Dimethylocta-(E)-2,6-dienyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-(2-hydroxyethyl)-1-azoniabicyclo[2.2.2]octane trifluoroacetate
(3R)-3-(2-Hydroxy-2, 2-dithien-2-ylacetoxy)-1-(3-hydroxypropyl)-1-azoniabicyclo[2.2.2]octane trifluoroacetate
(3R)-1-(4-Hydroxybutyl)-3-(2-hydroxy-2,2-dithien-2-ylacetoxy)-1-azoniabicyclo[2.2.2]octane trifluoroacetate
(3R)-1-(2-Ethoxyethyl)-3-(2-hydroxy-2,2-dithien-2-ylacetoxy)-1-azoniabicyclo[2.2.2]octane bromide
(3R)-3-(2-Hydroxy-2,2-dithien-2-ylacetoxy)-1-[2-(2-hydroxyethoxy)ethyl]-1-azoniabicyclo[2.2.2]octane chloride
(3R)-3-(2-Hydroxy-2,2-dithien-2-ylacetoxy)-1-[2-(2-methoxyethoxy)ethyl]-1-azoniabicyclo[2.2.2]octane bromide
(3R)-3-(2-Hydroxy-2,2-dithien-2-yl-acetoxy)-1-oxiranylmethyl-1-azoniabicyclo[2.2.2]octane bromide
(3R)-1-(2-[1,3]Dioxolan-2-ylethyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-{2-[2-(2-hydroxyethoxy)-ethoxy]ethyl}-1-azoniabicyclo[2.2.2]octane trifluoroacetate
(3R)-1-(3-[1,3]Dioxolan-2-ylpropyl)-3-(2-hydroxy-2,2-dithien-2-ylacetoxy)-1-azoniabicyclo[2.2.2]octane trifluoroacetate
(3R)-1-(3-Ethoxycarbonylpropyl)-3-(2-hydroxy-2,2-dithien-2-ylacetoxy)-1-azoniabicyclo[2.2.2]octane trifluoroacetate
(3R)-1-(4-Ethoxycarbonylbutyl)-3-(2-hydroxy-2,2-dithien-2-ylacetoxy)-1-azoniabicyclo[2.2.2]octane trifluoroacetate
(3R)-1-(4-Acetoxybutyl)-3-(2-hydroxy-2,2-dithien-2-ylacetoxy)-1-azoniabicyclo[2.2.2]octane trifluoroacetate
(3R)-1-(3-Cyanopropyl)-3-(2-hydroxy-2,2-dithien-2-ylacetoxy)-1-azoniabicyclo[2.2.2]octane trifluoroacetate
(3R)-1-(4-Cyanobutyl)-3-(2-hydroxy-2,2-dithien-2-ylacetoxy)-1-azoniabicyclo[2.2.2]octane trifluoroacetate
(3R)-1-(6-Cyanohexyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-(4,4,4-trifluorobutyl)-1-azoniabicyclo[2.2.2]octane trifluoroacetate
(3R)-1-Allyl-3-(2-hydroxy-2-phenyl-2-thien-2-ylacetoxy)-1-azoniabicyclo[2.2.2]octane bromide
(3R)-1-Allyl-3-(2-fur-2-yl-2-hydroxy-2-phenylacetoxy)-1-azoniabicyclo[2.2.2]octane bromide
(3R)-1-Allyl-3-(2-fur-2-yl-2-hydroxy-2-thien-2-ylacetoxy)-1-azoniabicyclo[2.2.2]octane bromide
(3R)-1-Allyl-3-(9-methyl-9H-fluorene-9-carbonyloxy)-1-azoniabicyclo[2.2.2]octane bromide
(3R)-1-Allyl-3-(9-hydroxy-9H-fluorene-9-carbonyloxy)-1-azoniabicyclo[2.2.2]octane bromide
(3R)-3-(9-Hydroxy-9H-fluorene-9-carbonyloxy)-1-(4-methylpent-3-enyl)-1-azoniabicyclo[2.2.2]octane bromide
(3R)-1-Heptyl-3-(9-hydroxy-9H-fluorene-9-carbonyloxy)-1-azoniabicyclo[2.2.2]octane bromide
(3R)-3-(9-Hydroxy-9H-fluorene-9-carbonyloxy)-1-oxiranylmethyl-1-azoniabicyclo[2.2.2]octane bromide
(3R)-3-(9-Hydroxy-9H-fluorene-9-carbonyloxy)-1-[2-(2-methoxyethoxy)ethyl]-1-azoniabicyclo[2.2.2]octane bromide
(3R)-1-(2-[1,3]Dioxolan-2-ylethyl)-3-(9-hydroxy-9H-fluorene-9-carbonyloxy)-1-azoniabicyclo[2.2.2]octane bromide
(3R)-1-Allyl-3-(9H-xanthene-9-carbonyloxy)-1-azoniabicyclo[2.2.2]octane bromide
(3R)-1-(4-Methylpent-3-enyl)-3-(9H-xanthene-9-carbonyloxy)-1-azoniabicyclo[2.2.2]octane bromide
(3R)-1-Allyl-3-(9-methyl-9H-xanthene-9-carbonyloxy)-1-azoniabicyclo[2.2.2]octane bromide
(3R)-1-Allyl-3-(9-hydroxy-9H-xanthene-9-carbonyloxy)-1-azoniabicyclo[2.2.2]octane bromide
(3R)-1-(3-Hydroxypropyl)-3-(9-hydroxy-9H-xanthene-9-carbonyloxy)-1-azoniabicyclo[2.2.2]octane bromide
(3R)-1-Allyl-3-(10,11-dihydro-5H-dibenzo[a,d]cycloheptene-5-carbonyloxy)-1-azoniabicyclo[2.2.2]octane bromide
(3R)-1-(4-Methylpent-3-enyl)-3-(10,11-dihydro-5H-dibenzo[a,d]cycloheptene-5-carbonyloxy)-1-azoniabicyclo[2.2.2]octane bromide
(3R)-1-Allyl-3-[(2*)-2-hydroxy-2,3-diphenylpropionyloxy]-1-azoniabicyclo[2.2.2]octane bromide
(3R)-3-[(2*)-2-Hydroxy-2,3-diphenylpropionyloxy)]-1-(4-methylpent-3-enyl)-1-azoniabicyclo[2.2.2]octane bromide
(3R)-1-Allyl-3-(2-hydroxy-2-thien-2-ylpent-4-enoyloxy)-1-azoniabicyclo[2.2.2]octane bromide
(3R)-1-(4-Methylpent-3-enyl)-3-(2-Hydroxy-2-thien-2-ylpent-4-enoyloxy)-1-azoniabicyclo[2.2.2]octane bromide
(3R)-1-Allyl-3-[(2S)-2-cyclopentyl-2-hydroxy-2-thien-2-ylacetoxy]-1-azoniabicyclo[2.2.2]octane bromide
(3R)-1-(4-Methylpent-3-enyl)-3-[(2S)-2-cyclopentyl-2-hydroxy-2-thien-2-ylacetoxy)-1-azoniabicyclo[2.2.2]octane bromide
(3R)-1-Allyl-3-[(2R)-2-cyclopentyl-2-hydroxy-2-thien-2-ylacetoxy)-1-azoniabicyclo[2.2.2]octane bromide
(3R)-3-[(2S)-2-Cyclopentyl-2-hydroxy-2-thien-2-ylacetoxy]-1-(2-hydroxyethyl)-1-azoniabicyclo[2.2.2]octane bromide
(3R)-3-[(2S)-2-Cyclopentyl-2-hyd roxy-2-thien-2-ylacetoxy]-1-(3-hydroxypropyl)-1-azoniabicyclo[2.2.2]octane bromide
(3R)-3-[(2S)-2-Cyclopentyl-2-hydroxy-2-th ien-2-ylacetoxy]-1-(4-hydroxybutyl)-1-azoniabicyclo[2.2.2]octane bromide
(3R)-3-[(2S)-2-Cyclopentyl-2-hydroxy-2-thien-2-ylacetoxy]-1-[2-(2-hydroxyethoxy)ethyl]-1-azoniabicyclo[2.2.2]octane bromide
(3R)-3-[(2S)-2-Cyclopentyl-2-hydroxy-2-thien-2-ylacetoxy]-1-(6-hydroxyhexyl)-1-azoniabicyclo[2.2.2]octane bromide
(3R)-3-[(2S)-2-Cyclopentyl-2-hydroxy-2-thien-2-ylacetoxy]-1-(5-hydroxypentyl)-1-azoniabicyclo[2.2.2]octane bromide
(3R)-3-[(2S)-2-Cyclopentyl-2-hydroxy-2-thien-2-ylacetoxy]-1-[3-(4-hydroxypiperidin-1-yl)propyl]-1-azoniabicyclo[2.2.2]octane bromide
(3R)-3-[(2S)-2-Cyclopentyl-2-hydroxy-2-thien-2-ylacetoxy]-1-[4-(4-hydroxypiperidin-1-yl)butyl]-1-azoniabicyclo[2.2.2]octane bromide
(3R)-3-(9,10-Dihydroanthracene-9-carbonyloxy)-1-(2-hydroxyethyl)-1-azoniabicyclo[2.2.2]octane bromide
(3R)-1-(2-Hydroxyethyl)-3-(9H-xanthene-9-carbonyloxy)-1-azoniabicyclo[2.2.2]octane bromide (3R)-1-(2-Hydroxyethyl)-3-(9-hydroxy-9H-xanthene-9-carbonyloxy)-1-azoniabicyclo[2.2.2]octane bromide
(3R)-1-(2-Hydroxyethyl)-3-(9-hydroxy-9H-fluorene-9-carbonyloxy)-1-azoniabicyclo[2.2.2]octane bromide
(3R)-1-(2-Hydroxyethyl)-3-(9-methyl-9H-fluorene-9-carbonyloxy)-1-azoniabicyclo[2.2.2]octane bromide
(* Configuration not assigned)

Of outstanding interest are the compounds:
1-Heptyl-(3R)-3-(2-hydroxy-2,2-dithien-2-ylacetoxy)-1-azoniabicyclo[2.2.2]octane bromide
1-Allyl-(3R)-3-(9-methyl-9H-xanthene-9-carbonyloxy)-1-azoniabicyclo[2.2.2]octane bromide
1-Allyl-(3R)-3-(9-hydroxy-9H-xanthene-9-carbonyloxy)-1-azoniabicyclo[2.2.2]octane bromide

In another aspect the present invention also provides processes for preparing compounds of formula (I).

The new quaternary ammonium derivatives of general formula (I), may be prepared, as illustrated in the following scheme, by reaction of an alkylating agent of formula (II) with compounds of general formula (III) using two possible methods (a) or (b), described in detail in the experimental section. Method (b) involves the use of solid phase extraction techniques that allow the parallel preparation of several compounds.

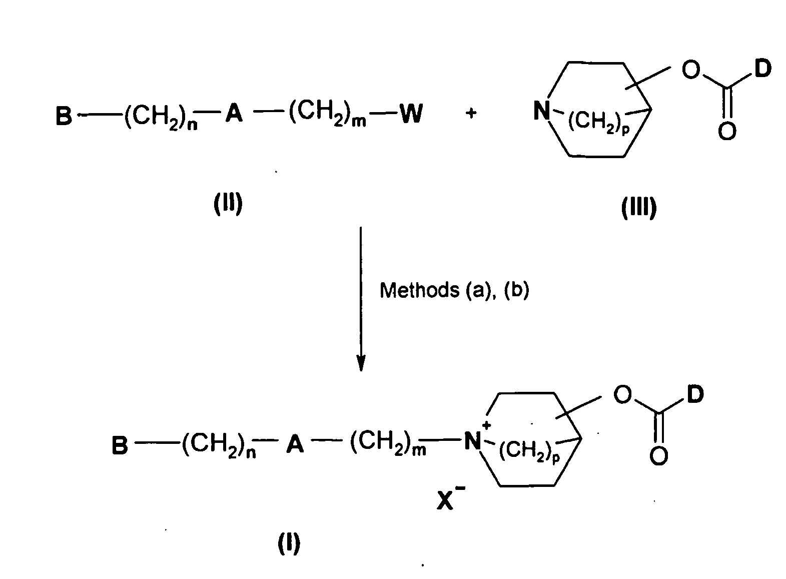

In formula (II), W represents any suitable leaving group, preferably a group X⁻ as defined above for the compounds of general formula (I). When W is a leaving group other than X⁻, the quaternary ammonium salt of formula (I) is produced from the product of method (a) or (b) by an exchange reaction according to standard methods to replace the anion W with the desired anion X⁻.

Those compounds of general formula (II) which are not commercially available may be prepared according to standard methods. For example, some examples of compounds of formula (II) wherein A = -O-, -S-, -NR³-, may be obtained by reaction of the corresponding alcohol, thiol or amine derivative, or its sodium or potassium salt, with an alkylating agent of general formula Y-(CH₂)ₘ-W, wherein W is as defined above; most preferably W is a halogen atom and Y is a halogen atom or a sulphonate ester. In other examples, compounds of general formula (II) may be synthesised from the corresponding alcohol derivative of general formula (IV) by methods known in the art.

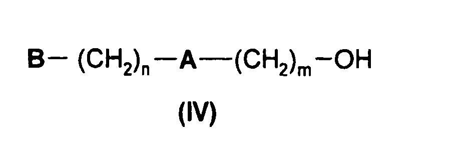

The compounds of formula (I) and (III) may have one or more asymmetric carbons. All possible stereoisomers, single isomers and mixtures of isomers are also included within the scope of the present invention. The diastereomers of the compounds may be separated by conventional methods, for example by chromatography or crystallisation.

Compounds of formula (III) may be prepared by three different methods, (c), (d), and (e) as is described in the following scheme and detailed in the experimental section.

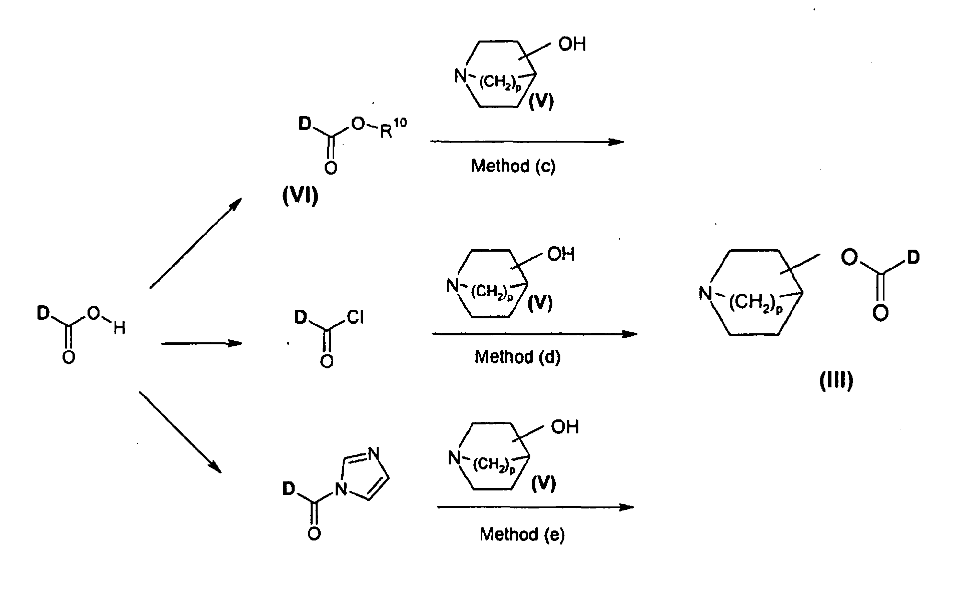

In the compounds of formula (VI) described in method (c), R¹⁰ is a lower alkyl group.

The following compounds of Formula (V) are described in the literature:
4-hydroxy-1-azabicyclo[2.2.1]heptane, described in WO 93/15080
4-hydroxy-1-azabicyclo[2.2.2]octane, described in Grob, C.A. et.al. Helv.Chim.Acta (1958), 41, 1184-1190
(3R)-3-hydroxy-1-azabicyclo[2.2.2]octane or (3S)-3-hydroxy-1-azabicyclo[2.2.2]octane described in Ringdahl, R. Acta Pharm Suec. (1979), 16, 281-283 and commercially available from CU Chemie Uetikon GmbH.

Some compounds of general formula (III) where **D** is a group of formula i), R⁵ and R⁶ are as described above and R⁷ is a hydroxy group, may also be prepared from the glyoxalate esters of general formula (VII) by reaction with the corresponding organometallic derivative as is described in the following scheme and detailed in the experimental section (method (f)).

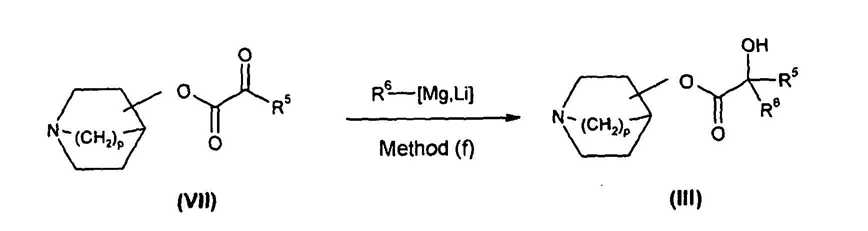

Compounds of formula (VII) may be prepared from the corresponding glyoxylic acids following the standard methods (c), (d) and (e) or as is described in WO 01/04118 and WO 92/04346.

As is described in the following scheme, compounds of formula (III) where R⁷ is a -CH₂OH group, may also be prepared from the corresponding compound of formula (III), wherein R⁷ is an H atom, by reaction with formaldehyde in basic conditions (see method (g), WO 93/06098 and WO02/053564).

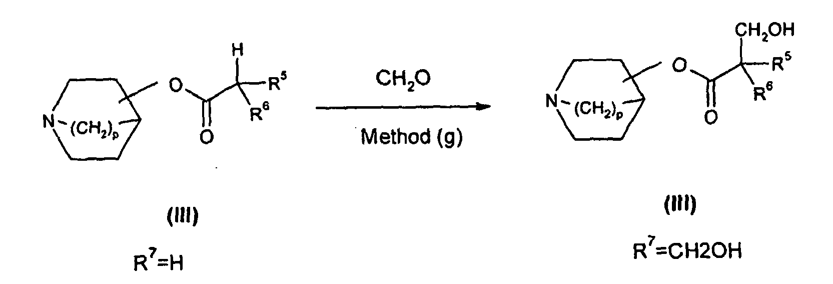

The structures of the prepared compounds were confirmed by ¹H-NMR and MS. The NMR were recorded using a Varian 300 MHz instrument and chemical shifts are expressed as parts per million (δ) from the internal reference tetramethyl silane. Their purity was determined by HPLC, using reverse phase chromatography on a Waters instrument. Molecular ions were obtained by electrospray ionization mass spectrometry on a Hewlett Packard instrument.

Optical rotations were measured using a PERKIN-ELMER 241 MC polarimeter.

The following examples are intended to illustrate but not to limit the experimental procedures that have been described before.

### Method (a)

### Example 37

### (3R)-1-Heptyl-3-(9-hydroxy-9H-fluorene-9-carbonyloxy)-1-azoniabicyclo[2.2.2]octane bromide

250 mg (0.75 mmol) of 9-Hydroxy-9H-fluorene-9-carboxylic acid (3R)-1-azabicyclo[2.2.2]oct-3-yl ester (Intermediate 1-3) were dissolved in 4 ml of acetonitrile and 6 ml of CHCl₃. To this solution was added 0.59 ml (0.67g, 3.75 mmol) of 1-bromoheptane. After stirring for 72 h at room temperature under a N₂ atmosphere, solvents were evaporated. Ether was added and the mixture stirred. The solid obtained was washed several times with ether and filtered. The yield was 330 mg (85.5%) of the title compound.
m.p.: 214.9-216.6°C
MS [M-Br]⁺: 434
¹H-NMR (300 MHz, DMSO-d₆) δ ppm: 0.88 (t, 3 H), 1.12-1.70 (m, 12 H), 1.70-1.93 (m, 2 H), 2.04 (m, 1 H), 2.78 (m, 1 H), 2.96-3.18 (m, 3 H), 3.18-3.48 (m, 3 H), 3.77 (m, 1 H), 5.01 (m, 1 H), 6.84 (s, 1 H, OH), 7.34 (m, 2 H), 7.45 (m, 2 H), 7.61 (m, 2 H), 7.84 (d, 2 H).

### Example 39

### (3R)-3-(9-Hydroxy-9H-fluorene-9-carbonyloxy)-1-[2-(2-methoxyethoxy)ethyl]-1-azoniabicyclo[2.2.2]octane bromide

0.5 g (0.0015 mol) of 9-Hydroxy-9H-fluorene-9-carboxylic acid (3R)-1-azabicyclo[2.2.2]oct-3-yl ester (Intermediate 1-3) were dissolved in 10 ml of acetonitrile and 15 ml of CHCl₃. To this solution 1.02 ml (1.37 g, 0.0075 mol) of 1-bromo-2-(2-methoxyethoxy)ethane were added and the mixture was stirred at room temperature during 96 hours. After this time a new portion (0.2 ml, 0.27 g, 0.0015 mol) of 1-bromo-2-(2-methoxyethoxy)ethane was added and the mixture stirred at room temperature during 24 hours more. After this time solvents were evaporated. Ether was added and the mixture stirred to obtain a solid, the solvent was extracted and more ether was added. This procedure was repeated several times in order to eliminate the residual alkylating agent. Finally the suspension was filtered and the solid obtained washed with ether and dried. The yield was 610 mg (78.2%) of the title compound.
m.p.: 194°C
MS [M-Br]⁺: 438
¹H-NMR (300 MHz, DMSO-d₆) δ ppm: 1.44 (m, 1 H), 1.64 (m, 1 H), 1.85 (m, 2 H), 2.09 (m, 1 H), 2.93 (m, 1 H), 3.18-3.28 (m, 1 H), 3.26 (s, 3 H), 3.30-3.60 (m, 9 H), 3.73 (m, 2 H), 3.88 (m, 1 H), 5.00 (m, 1 H), 6.83 (s, 1 H, OH), 7.35 (m, 2 H), 7.45 (m, 2 H), 7.61 (m, 2 H), 7.83 (d, 2 H).

### Example 43

### (3R)-1-Allyl-3-(9-methyl-9H-xanthene-9-carbonyloxy)-1-azoniabicyclo[2.2.2]octane bromide

1.05 g (3 mmol) of 9-Methyl-9H-xanthene-9-carboxylic acid (3R)-1-azabicyclo[2.2.2]oct-3-yl ester were dissolved in 15 ml of THF and 0.544 g (4.5 mmol) of 3-bromoprop-1-ene (allyl bromide) were added. The mixture was refluxed during 4 hours and allowed to continue stirring at room temperature for 15 hours. After this time the solvent was concentrated until % part of the initial volume. Ether was added and the mixture stirred during 20 min to obtain a solid, the solvent was extracted and more ether was added. This procedure was repeated several times in order to eliminate the residual alkylating agent. Finally the suspension was filtered and the solid obtained washed with ether and dried. The yield was 1.04 g (73.8%) of the title compound.
m.p.: 64.3-67.8°C
MS [M-Br]⁺ : 390
¹H-NMR (300 MHz, DMSO-d₆) δ ppm: 1.35 (m, 1 H), 1.65 (m, 1 H), 1.73-1.91 (m, 2 H), 1.89 (s, 3 H), 2.11 (m, 1 H), 2.75 (m, 1 H), 3.00 (m, 1 H), 3.15-3.45 (m, 3 H), 3.70-3.90 (m, 3 H), 5.03 (m, 1 H), 5.50-5.65 (m, 2 H), 5.86 (m, 1 H), 7.12-7.19 (m, 4 H), 7.35 (m, 2 H), 7.42 (m, 2 H).

### Method (b)

### Example 29

### (3R)-1-(6-Cyanohexyl)-3-(2-hydroxy-2,2-dithien-2-ylacetoxy)-1-azoniabicyclo[2.2.2]octane trifluoroacetate

60 mg (0.17 mmol) of 2-Hydroxy-2,2-dithien-2-ylacetic acid (3R)-1-azabicyclo[2.2.2]oct-3-yl ester were dissolved in 0.7 ml of DMSO. To this solution 161 mg (0.128 ml, 0.85 mmol) of 7-bromoheptanenitrile dissolved in 0.3 ml of DMSO were added. After stirring overnight at room temperature, the mixture was purified by solid phase extraction with a cation exchange Mega Bond Elut cartridge, previously conditioned at pH = 7.5 with 0.1 M NaH₂PO₄ buffer. The reaction mixture was applied to the cartridge an washed first with 2 ml of DMSO and then three times with 5 ml of CH₃CN, rinsing away all the starting materials. The ammonium derivative was eluted with 5 ml of 0.03 M TFA solution in CH₃CN:CHCl₃ (2:1). This solution was neutralized with 300 mg of poly(4-vinylpyridine), filtered and evaporated to dryness. The yield was 19.1 mg (19.6%) of the title compound.
MS [M-CF3COO]⁺: 459

### Method (c)

Methyl ester derivatives of general formula (VI) may be prepared by standard methods of esterification from the corresponding carboxylic acid or according to procedures described in literature: FR 2012964; Larsson, L. et al., Acta Pharm, Suec. (1974), 11 (3), 304-308; Nyberg, K. et al., Acta Chem. Scand. (1970), 24, 1590-1596; Cohen, V.I. et al., J. Pharm. Sciences (1992), 81, 326-329; WO 01/04118, WO 02/053564 and references cited therein.

### Intermediate 1-1

### Preparation of 2,2-Dithien-2-ylacetic acid (3R)-1-azabicyclo[2.2.2]oct-3-yl ester

1.67 g (0.007 mol) of 2,2-Dithien-2-ylacetic acid methyl ester were dissolved in 40 ml of toluene. To this solution were added 1.04 g (0.0082 mol) of (3R)-3-hydroxy-1-azabicyclo[2.2.2]octane and 0.14 g (0.0035 mol) of HNa (60% dispersion mineral oil). The mixture was refluxed during 10 min and, after this time, refluxed with continuous removal of distillate with replacement with fresh toluene when necessary for two hours. The cooled mixture was extracted with 2N HCI acid, the aqueous layer washed with ethyl acetate, basified with K₂CO₃ and extracted with CHCI₃. The organic layer was washed with a small volume of water, dried over Na₂SO₄ and evaporated. The oil obtained (2 g) was purified by chromatography on silica gel eluting with CHCl₃/MeOH/NH₄OH 95:5:0.5. Appropriate fractions were combined and evaporated to obtain the title product as an oil (0.82 g, 35%).
This product was solidified by formation of the fumarate salt. A portion of 0.43 g (0.00128 mol) of the oil obtained (free base) was treated with fumaric acid in acetone/diethyl ether to obtain a solid which was filtered and washed with ether. The yield was 0.44 g of the fumarate salt. 2,2-Dithien-2-ylacetic acid (3R)-1-azabicyclo[2.2.2]oct-3-yl ester fumarate salt:
m.p.:122°C
MS [M free base+1]⁺: 334
¹H-NMR (300 MHz, DMSO-d₆) δ ppm: 1.44 (m, 1 H), 1.64 (m, 3 H), 2.03 (m, 1 H), 2.62-2.98 (m, 5 H), 3.30 (m, 1 H), 4.89 (m, 1 H), 5.84 (s, 1 H), 6.54 (s, 2 H), 7.01 (m, 2 H), 7.09 (m, 2 H), 7.48 (m, 2 H).

2,2-Dithien-2-ylacetic acid methyl ester was prepared by reduction of 2-Hydroxy-2,2-dithien-2-ylacetic acid methyl ester following the method described in F. Leonard; I. Ehranthal, J. Am. Chem. Soc, Vol 73, pag 2216-2218, (1951).

### Intermediate 1-2

### Preparation of 2,2-Dithien-2-ylpropionic acid (3R)- 1-azabicyclo[2.2.2]oct-3-yl ester

0.86 g (0.0034 mol) of 2,2-Dithien-2-ylpropionic acid methyl ester were dissolved in 25 ml of toluene. To this solution were added 0.51 g (0.004 mol) of (3R)-3-hydroxy-1-azabicyclo[2.2.2]octane and 0.055 g (0.0014 mol) of HNa (60% dispersion mineral oil). The mixture was refluxed during 10 minutes and, after this time, refluxed with continuous removal of distillate with replacement with fresh toluene when necessary for 1.5 hours. The cooled mixture was extracted with 2N HCl acid, the aqueous layer washed with ethyl acetate, basified with K₂CO₃ and extracted with CHCI₃. The organic layer was washed with a small volume of water, dried over Na₂SO₄ and evaporated. The yield was 1.11 g of the title product as an oil (94.07%).
GC/MS [M]⁺: 347
This product was solidified by formation of the oxalate salt: 0.25 g of the free base (0.00072 mol) were treated with oxalic acid (0.065 g, 0.00072 mol) in acetone/diethyl ether. A solid was obtained which was filtered and washed with ether. The yield was 0.25 g (79.4%).
2,2-Dithien-2-ylpropionic acid (3R)- 1-azabicyclo[2.2.2]oct-3-yl ester oxalate salt:
m.p.: 126.7-128.6°C
¹H-NMR (300 MHz, DMSO-d₆) δ ppm: 1.61 (m, 2 H), 1.83 (m, 2 H), 2.08 (s, 3 H), 2.19 (m, 1 H), 2.88 (m, 1 H), 2.95-3.28 (m, 4 H), 3.61 (m, 1 H), 5.09 (m, 1 H), 7.01 (m, 2 H), 7.07 (m, 2 H), 7.51 (m, 2 H), 9.15 (br. s., 2 H).

2,2-Dithien-2-ylpropionic acid methyl ester was prepared by a standard method of esterification from 2,2-Dithien-2-ylpropionic acid, prepared as described in M. Sy et al; Bull. Soc. Chim. Fr.; Vol 7, 2609-2611, (1957).

### Intermediate 1-3

### Preparation of 9-Hydroxy-9H-fluorene-9-carboxylic acid (3R)-1-azabicyclo[2.2.2]oct-3-yl ester

1.0 g (0.0042 mol) of 9-Hydroxy-9H-fluorene-9-carboxylic acid methyl ester were dissolved in 25 ml of toluene. To this solution were added 0.67 g (0.0053 mol) of (3R)-3-hydroxy-1-azabicyclo[2.2.2]octane and 0.064 g (0.0016 mol) of HNa (60% dispersion in mineral oil). The mixture was refluxed during 15 min and, after this time, refluxed with continuous removal of distillate with replacement with fresh toluene when necessary for 1.5 hours. The cooled mixture was extracted with 2N HCl acid, the aqueous layer washed with diethyl ether, basified with K₂CO₃ and extracted with CHCl₃. The organic layer was washed with water, dried over Na₂SO₄ and evaporated. The solid obtained was treated with diethyl ether and filtered. The product obtained was recrystallised from a mixture of CHCl₃/diisopropyl ether filtered and washed with diisopropyl ether. The yield was 0.75 g of the title product. (53.2%).
m.p.: 217°C
MS [M+1]⁺ : 336
¹H-NMR (300 MHz, DMSO-d₆) δ ppm: 1.00 (m, 2 H), 1.25-1.50 (m, 2 H), 1.60 (m, 1 H), 2.0-2.16 (m, 2 H), 2.37-2.56 (m, 3 H), 2.91 (m, 1 H), 4.57 (m, 1 H), 6.74 (br. s., 1 H, OH), 7.31 (m, 2 H), 7.42 (m, 2 H), 7.51 (m, 2 H), 7.81 (m, 2 H).

9-Hydroxy-9H-fluorene-9-carboxylic acid methyl ester was prepared from 9-Hydroxy-9H-fluorene-9-carboxylic acid (commercially available) using a standard method of esterification.

The following compounds of formula (III) have also been prepared according to method (c):
2-Hydroxy-2,2-dithien-2-ylacetic acid (3R)-1-azabicyclo[2.2.2]oct-3-yl ester,
2-Fur-2-yl-2-hydroxy-2-phenylacetic acid (3R)-1-azabicyclo[2.2.2]oct-3-yl ester,
2-Fur-2-yl-2-hydroxy-2-thien-2-ylacetic acid (3R)-1-azabicyclo[2.2.2]oct-3yl ester,
9-Methyl-9H-fluorene-9-carboxylic acid (3R)-1-azabicyclo[2.2.2]oct-3-yl ester,
9-Methyl-9H-xanthene-9-carboxylic acid (3R)-1-azabicyclo[2.2.2]oct-3-yl ester,
9-Hydroxy-9H-xanthene-9-carboxylic acid (3R)-1-azabicyclo[2.2.2]oct-3-yl ester,
2-Hydroxy-2-thien-2-ylpent-4-enoic acid (3R)-1-azabicyclo[2.2.2]oct-3-yl ester,
(2S)-2-Cyclopentyl-2-hydroxy-2-thien-2-yl acetic acid (3R)-1-azabicyclo[2.2.2]oct-3-yl ester and
(2R)-2-Cyclopentyl-2-hydroxy-2-thien-2-yl acetic acid (3R)-1-azabicyclo[2.2.2]oct-3-yl ester (the two last compounds have also been prepared by method (f)).

### Method (d)

### Intermediate I-4

### Preparation of 10,11-Dihydro-5H-dibenzo[a,d]cycloheptene-5-carboxylic acid (3R)-1-azabicyclo[2.2.2]oct-3-yl ester

2.15 g of 10,11-Dihydro-5H-dibenzo[a,d]cycloheptene-5-carboxylic acid (9.0 mmol) were dissolved in 40 ml of CHCl₃ (ethanol free). The solution was cooled at 0°C and 0.86 ml of oxalyl chloride (9.9 mmols) and a drop of DMF were added. The mixture was stirred and allowed warm to room temperature. After an hour at this temperature the solvents were evaporated and the residue was dissolved in CHCl₃ and evaporated again. This procedure was repeated two times. The obtained oil was dissolved in 20 ml of toluene and added to a solution of 1.26 g (9.9 mmol) of (3R)-3-hydroxy-1-azabicyclo[2.2.2]octane in 40 ml of hot toluene. The reaction mixture was refluxed for 2 hours. After cooling the mixture was extracted with 2N HCl acid. The aqueous layer was basified with K₂CO₃ and extracted with CHCl₃. The organic layer was dried over Na₂SO₄ and evaporated to dryness. The residue was purified by column chromatography (silica gel, CHCl₃:MeOH:NH₄OH, 95:5:0.5). The yield was 1.5 g (48%) of the title product.
m.p.: 112-113°C
CG/MS [M]⁺: 347
¹H-NMR (300 MHz, CDCl₃) δ ppm : 1.10-1.35 (m, 2 H), 1.40-1.52 (m, 1 H), 1.52-1.68 (m, 1 H), 1.90 (m, 1 H), 2.40-2.60 (m, 2 H), 2.60-2.77 (m, 3 H), 2.83-2.96 (m, 2 H), 3.07-3.19 (m, 1 H), 3.25-3.40 (m, 2 H), 4.80 (m, 2 H), 7.10-7.30 (m, 8 H).
10,11-Dihydro-5H-dibenzo[a,d]cycloheptene-5-carboxylic acid was prepared as described in Kumazawa T. et al., J. Med. Chem., (1994), 37, 804-810.

The following compound of formula (III) has also been prepared according to method (d) from the corresponding carboxylic acid:
9H-Xanthene-9-carboxylic acid (3R)-1-azabicyclo[2.2.2]oct-3-yl ester.

### Method (e)

### Intermediate I-5

### Preparation of (2R)-2-Cyclohexyl-2-hydroxy-2-phenylacetic acid 1-azabicyclo[2.2.2]oct-4-yl ester.

660 mg (0.00282 mol) of (2R)-2-Cyclohexyl-2-hydroxy-2-phenylacetic acid (obtained as is described in WO 02/053564) were dissolved in 9 ml of DMF. This solution was stirred at room temperature and 548 mg (0.00338 mol) of 1,1'-carbonyldiimidazole were added. The reaction was monitored by TLC (CHCl₃/MeOH/AcOH 70:30:2) following the formation of the imidazolide. After 1 hour the reaction was complete. The reaction mixture was cooled to 0°C and 394 mg (0.0031 mol) of 4-hydroxy-1-azabicyclo[2.2.2]octane and 104 mg (0.00259 mol) of HNa (60% dispersion in mineral oil) were added. After 44 h of stirring at room temperature the reaction mixture was treated with water and extracted three times with diethyl ether. The organic layers were combined, washed with brine and dried over anhydrous magnesium sulphate. The solvent was evaporated and the residue was purified by silica gel column chromatography eluting with CHCl₃ to CHCl₃/MeOH 15:1. The yield was 300 mg (31 %) of the title product :
[α]²²_{D} = -27.6 ° (c=1, CHCl₃)
MS: [M+1]⁺: 344
¹H-NMR (300 MHz, CDCl₃) δ ppm: 1.0-1.55 (m, 7H), 1.55-1.75 (m, 2H), 1.75-1.85 (m, 1H), 1.85-2.05 (m, 6H), 2.10-2.22 (m, 1H), 2.90-3.10 (m, 6H), 3.60-3.80 (bs, 1H, OH), 7.20-7.40 (m, 3H), 7.57-7.67 (m, 2H).

### Method (f)

### Intermediate 1-6

### Preparation of 2-hydroxy-2,3-diphenylpropionic acid (3R)-1-azabicyclo[2.2.2]oct-3-yl ester

Benzylmagnesium chloride, 0.00386 mol (1.93 ml of a solution 2M in THF), was added to a solution of 1 g (0.00385 mol) of 2-oxo-2-phenylacetic acid (3R)-1-azabicyclo[2.2.2]oct-3-yl ester, dissolved in 8 ml of THF, at -70°C under a N₂ atmosphere. The mixture was stirred at this temperature for 10 minutes, then warmed to room temperature and diluted with 4 ml more of THF. After 1 h, the reaction mixture was treated with 10% K₂CO₃ solution and extracted twice with ethyl acetate. The organic phases were combined and dried over Na₂SO₄. After removal of the solvent, the oil obtained was partitioned between HCl 2N and diethyl ether. The aqueous phase was basified with K₂CO₃ an extracted with CHCl₃. The organic solution was washed with water, dried over Na₂SO₄ and solvent was evaporated to yield 1.2 g of an oil.
This reaction process was reproduced starting from 2 g (0.0077 mol) of 2-oxo-2-phenylacetic acid (3R)-1-azabicyclo[2.2.2]oct-3-yl ester and 0.0077 mol of benzylmagnesium chloride (3.85 ml of a solution 2M in THF), to obtain 2.91g of the final oil.
The total amount of product (4.11 g) was purified by column chromatography (silica gel) eluting with CHCl₃/MeOH/NH₄OH 99:1:0.1→ 95:5:0.5. Appropriate fractions were combined to give 1.86 g of a pure product as a solid mixture of diastereomers 1-6a and 1-6b, which were separated by maceration using diethyl ether / diisopropyl ether.

### Intermediate 1-6a

### Preparation of (2*)-2-Hydroxy-2,3-diphenylpropionic acid (3R)-1-azabicyclo[2.2.2]oct-3-yl ester (diastereomer 1, first diastereomer obtained)

The 1.86 g of the mixture of diastereomers (I-6) were treated with a mixture of diethyl ether / diisopropyl ether and filtered to give a solid identified as a pure diastereomer.
The yield was 0.87 g (42.6% based on single isomer),
m.p.: 132°C.
MS [M+1]⁺ : 352
¹H-NMR (300 MHz, CDCl₃) δ ppm : 1.30-1.60 (m, 2 H), 1.60-1.90 (m, 2 H), 2.05 (m, 1 H), 2.20-2.35 (m, 1 H), 2.50-2.90 (m, 4 H), 3.0-3.15 (m, 1 H), 3.25 and 3.60 (dd, 2 H), 3.70 (br.s., 1 H, OH), 4.70-4.80 (m, 1 H), 7.15-7.45 (m, 8 H), 7.65-7.75 (m, 2 H).

### Intermediate 1-6b

### Preparation of (2*)-2-Hydroxy-2,3-diphenylpropionic acid (3R)-1-azabicyclo[2.2.2]oct-3-yl ester (diastereomer 2, second diastereomer obtained).

The mother liquors of filtration of the first diastereomer were enriched in the second diastereomer. After evaporation of the solvents, 0.55 g of the residue were treated with diethyl ether and filtered to give a solid identified as the pure second diastereomer.
The yield was 0.23 g (11.2% based on single isomer),
m.p.: 107°C.
MS [M+1]⁺ : 352
¹H-NMR (300 MHz, CDCl₃) δ ppm : 1.20-1.35 (m, 1 H), 1.35-1.55 (m, 2 H), 1.55-1.70 (m, 1 H), 1.80-1.95 (m, 1 H), 2.55-2.90 (m, 5 H), 3.10-3.20 (m, 1 H), 3.25 and 3.60 (dd, 2 H), 3.80 (br.s., 1 H, OH), 4.65-4.80 (m, 1 H), 7.20-7.50 (m, 8 H), 7.65-7.75 (m, 2 H).
((*): Configuration not assigned, either the (2R)- or the (2S)- isomers of the above compounds may be obtained)

2-oxo-2-phenylacetic acid (3R)-1-azabicyclo[2.2.2]oct-3-yl ester was prepared as is described in WO 92/04346.

The following compounds of formula (III) have also been prepared following method (f):
(2S)-2-Cyclopentyl-2-hydroxy-2-thien-2-yl acetic acid (3R)-1-azabicyclo[2.2.2]oct-3-yl ester,
(2R)-2-Cyclopentyl-2-hydroxy-2-thien-2-yl acetic acid (3R)-1-azabicyclo[2.2.2]oct-3-yl ester (compounds also prepared by method (c)) and 2-Hydroxy-2-phenyl-2-thien-2-ylacetic acid (3R)-1-azabicyclo[2.2.2]oct-3-yl ester.

Other carboxylic acids of formula D-C(O)OH, whose preparation (or the syntheses of their derivatives methyl ester, chloride or imidazolide) haven't been described in methods (c), (d), (e), and that are not commercially available, could be prepared as is described in the following references:
FR 2012964
M.A. Davis et al; J. Med. Chem. (1963), 6, 513-516.
T. Kumazawa et al; J.Med. Chem, (1994), 37(6), 804-810.
M.A. Davis et al; J. Med. Chem., (1964), Vol(7), 88-94.
Sestanj, K; Can. J. Chem., (1971), 49, 664-665.
Burtner, R. ; J. Am. Chem. Soc., (1943), 65,1582-1585
Heacock R.A. et al; Ann. Appl. Biol., (1958), 46(3), 352-365.
Rigaudy J. et.al; Bull. Soc. Chim. France, (1959), 638-43.
Ueda I. et al; Bull. Chem. Soc. Jpn; (1975), 48 (8), 2306-2309.
E.L. May et.al.; J. Am. Chem. Soc., (1948), 70, 1077-9.
G.W. Moersch et al; Synthesis, (1971), 647-648;
A. Waldemar et al; J.Org. Chem., (1977), Vol 42 (1), 38-40.
WO 01/04118 and WO 02/053564.

Also included within the scope of the present invention are pharmaceutical compositions which comprise, as the active ingredient, at least one quinuclidine derivative of general formula (I) in association with a pharmaceutically acceptable carrier or diluent. Preferably the composition is made up in a form suitable for oral administration.

The pharmaceutically acceptable carrier or diluents which are mixed with the active compound or compounds, to form the composition of this invention are well-known *per se* and the actual excipients used depend *inter alia* on the intended method of administration of the composition.

Compositions of this invention are preferably adapted for oral administration. In this case, the composition for oral administration may take the form of tablets, film-coated tablets, liquid inhalant, powder inhalant and inhalation aerosol; all containing one or more compounds of the invention; such preparations may be made by methods well-known in the art.

The diluents which may be used in the preparations of the compositions include those liquid and solid diluents which are compatible with the active ingredient, together with colouring or flavouring agents, if desired. Tablets or film-coated tablets may conveniently contain between 1 and 500 mg, preferably from 5 to 300 mg of active ingredient. The inhalant compositions may contain between 1 µg and 1,000 µg, preferably from 10 µg to 800 µg of active ingredient. In human therapy, the dose of the compound of general formula (I) depend on the desired effect and duration of treatment; adult doses are generally between 3 mg and 300 mg per day as tablets and 10 µg and 800 µg per day as inhalant composition.

### Pharmacological Action

The results on human muscarinic receptors binding and in the test on bronchospasm in guinea pig, were obtained as described below.

### Human muscarinic receptor studies.

The binding of [3H]-NMS to human muscarinic receptors was performed according to Waelbroek et al (1990), Mol. Pharmacol., 38: 267-273.Assays were carried out at 25°C. Membrane preparations from stably transfected Chinese hamster ovary-K1 cells (CHO) expressing the genes for the human muscarinic receptors M3 were used.

For determination of IC₅₀, membrane preparations were suspended in DPBS to a final concentration of 89 µg/ml for the M3 subtype. The membrane suspension was incubated with the tritiated compound for 60 min. After incubation the membrane fraction was separated by filtration and the bound radioactivity determined. Non specific binding was determined by addition of 10⁻⁴ M atropine. At least six concentrations were assayed in duplicate to generate individual displacement curves.

Our results show that the compounds of the present invention have high affinities for muscarinic M3 receptors, preferably human muscarinic receptors. Preferred compounds of the present invention have an IC₅₀ (nM) value for M3 receptors of less than 50, preferably less than 25, more preferably less than 15 and most preferably less than 10, 8 or 5.

### Test on bronchospasm in guinea pig

The studies were performed according to H. Konzett and F. Rössler (1940), Arch. Exp. Path. Pharmacol. 195: 71-74. Aqueous solutions of the agents to be tested were nebulized and inhaled by anaesthetized ventilated male guinea pigs (Dunkin-Hartley). Bronchial response to intravenous acetylcholine challenge was determined before and after drug administration and the changes in pulmonary resistance at several time-points were expressed as percent of inhibition of bronchospasm.

The compounds of the present invention inhibited the bronchospasm response to acetylcholine with high potency and a long duration of action.

From the above described results one of ordinary skill in the art can readily understand that the compounds of the present invention have excellent antimuscarinic activity (M3) and thus are useful for the treatment of diseases in which the muscarinic M3 receptor is implicated, including respiratory disorders such as chronic obstructive pulmonary disease (COPD), bronchitis, bronchial hyperreactivity, asthma, cough and rhinitis; urological disorders such as urinary incontinence, pollakiuria, neurogenic or unstable bladder, cystospasm and chronic cystitis; gastrointestinal disorders such as irritable bowel syndrome, spastic colitis, diverticulitis and peptic ulceration; and cardiovascular disorders such as vagally induced sinus bradycardia

The present invention further provides a compound of formula (I) or a pharmaceutically acceptable composition comprising a compound of formula (I) for use in a method of treatment of the human or animal body by therapy, in particular for the treatment of respiratory, urological or gastrointestinal disease or disorder.

The present invention further provides the use of a compound of formula (I) or a pharmaceutically acceptable composition comprising a compound of formula (I) for the manufacture of a medicament for the treatment of a respiratory, urological or gastrointestinal disease or disorder.

Further, the compounds of formula (I) and pharmaceutical compositions comprising a compound of formula (I) can be used in a method of treating a respiratory, urological or gastrointestinal disease or disorder, which method comprises administering to a human or animal patient in need of such treatment an effective, non-toxic, amount of a compound of formula (I) or a pharmaceutical composition comprising a compound of formula (I).

Further, the compounds of formula (I) and pharmaceutical compositions comprising a compound of formula (I) can be used in combination with other drugs effective in the treatment of these diseases. The compounds of formula (I) may, for example be combined with β₂ agonists, steroids, antiallergic drugs, phosphodiesterase IV inhibitors and/or leukotriene D4 (LTD4) inhibitors, for simultaneous, separate or sequential use in the treatment of a respiratory disease.

### EXAMPLES OF COMPOUNDS OF FORMULA (I)

### Example 1

### (3R)-1-Allyl-3-(2,2-dithien-2-ylacetoxy)-1-azoniabicyclo[2.2.2]octane bromide

The title compound was synthesised according to methods c, and a from Intermediate I-1. The yield of the final step was 310 mg, 90.9%.
MS [M-Br]⁺ : 374
¹H-NMR (300 MHz, DMSO-d₆) δ ppm : 1.72-2.07 (m, 4 H), 2.28 (m, 1 H), 3.07-3.56 (m, 5 H), 3.78-3.99 (m, 3 H), 5.19 (m, 1 H), 5.52-5.68 (m, 2 H), 5.89 (s, 1 H), 5.98 (m, 1 H), 7.01 (m, 2 H), 7.12 (m, 2 H), 7.50 (m, 2 H).

### Example 2

### (3R)-3-(2,2-Dithien-2-ylacetoxy)-1-(4-methylpent-3-enyl)-1-azoniabicyclo[2.2.2]octane bromide

The title compound was synthesised according to methods c, and a from Intermediate I-1. The yield of the final step was 270 mg, 72.9%.
m.p.: 163.5-165.1°C
MS [M-Br]⁺: 416
¹H-NMR (300 MHz, DMSO-d₆) δ ppm : 1.62 (s, 3 H), 1.67 (s, 3 H), 1.73-2.03 (m, 4 H), 2.20-2.43 (m, 3 H), 3.05-3.46 (m, 6 H), 3.52 (m, 1 H), 3.92 (m, 1 H), 4.98 (m, 1 H), 5.18 (m, 1 H), 5.89 (s, 1 H), 7.01 (m, 2 H), 7.13 (m, 2 H), 7.50 (m, 2 H).

### Example 3

### (3R)-1-Allyl-3-(2,2-dithien-2-ylpropionyloxy)-1-azoniabicyclo[2.2.2]octane bromide

The title compound was synthesised according to methods c, and a from Intermediate I-2. The yield of the final step was 260 mg, 77.2%.
m.p.: 156-158°C
MS [M-Br]⁺: 388
¹H-NMR (300 MHz, DMSO-d₆) δ ppm : 1.65 (m, 1 H), 1.80 (m, 1 H), 1.95 (m, 2 H), 2.08 (s, 3 H), 2.28 (m, 1 H), 3.06 (m, 1 H), 3.25-3.52 (m, 4 H), 3.82-4.05 (m, 3 H), 5.20 (m, 1 H), 5.53-5.67 (m, 2 H), 5.97 (m, 1 H), 7.01 (m, 2 H), 7.10 (m, 2 H), 7.51 (m, 2 H).

### Example 4

### (3R)-1-(4-Methylpent-3-enyl)-3-(2,2-dithien-2-ylpropionyloxy)-1-azoniabicyclo[2.2.2]octane bromide

The title compound was synthesised according to methods c, and a from Intermediate 1-2. The yield of the final step was 380 mg, 100%.
m.p.: 130-131°C
MS [M-Br]⁺: 430
¹H-NMR (300 MHz, DMSO-d₆) δ ppm : 1.57-1.82 (m, 2 H), 1.62 (s, 3 H), 1.67(s, 3 H), 1.91 (m, 2 H), 2.07 (s, 3 H), 2.22-2.42 (m, 3 H), 3.10 (m, 1 H), 3.17 (m, 2 H), 3.25-3.42 (m, 3 H), 3.48 (m, 1 H), 3.92 (m, 1 H), 4.98 (m, 1 H), 5.19 (m, 1 H), 7.02 (m, 2 H), 7.09 (m, 2 H), 7.50 (m, 2 H).

### Example 5

### (3R)-3-(2-Hydroxy-2,2-dithien-2-ylacetoxy)-1-isopropyl-1-azoniabicyclo[2.2.2]octane trifluoroacetate

The title compound was synthesised according to methods c and b. The yield of the final step was 21.6 mg, 25.2%.
MS [M-CF3COO]⁺: 392

### Example 6

### (3R)-1-Cyclopropylmethyl-3-(2-hydroxy-2,2-dithien-2-ylacetoxy)-1-azoniabicyclo[2.2.2]octane trifluoroacetate

The title compound was synthesised according to methods c and b. The yield of the final step was 5.5 mg, 6.2%.
MS [M-CF3COO]⁺: 404

### Example 7

### (3R)-3-(2-Hydroxy-2,2-dithien-2-ylacetoxy)-1-isobutyl-1-azoniabicyclo[2.2.2]octane trifluoroacetate

The title compound was synthesised according to methods c and b. The yield of the final step was 25.0 mg, 28.3%.
MS [M-CF3COO]⁺ : 406

### Example 8

### (3R)-1-Heptyl-3-(2-hydroxy-2,2-dithien-2-ylacetoxy)-1-azoniabicyclo[2.2.2]octane bromide

The title compound was synthesised according to methods c and a. The yield of the final step was 490 mg, 66.6%.
m.p.: 134°C
MS [M-Br]⁺ : 448
¹H-NMR (300 MHz, CDCl₃) δ ppm: 0.86 (t, 3 H), 1.16-1.32 (m, 8 H), 1.60 (m, 2 H), 1.91 (m, 2 H), 2.05 (m, 2 H), 2.42 (m, 1 H), 3.32-3.48 (m, 2 H), 3.48-3.80 (m, 5 H), 4.24 (m, 1 H), 5.28 (m, 1 H), 5.98 (s, 1 H, OH), 6.95 (m, 2 H), 7.17 (m, 1 H), 7.22-7.28 (m, 3 H).

### Example 9

### (3R)-1-Cyclohexylmethyl-3-(2-hydroxy-2,2-dithien-2-ylacetoxy)-1-azonabicyclo[2.2.2]octane trifluoroacetate

The title compound was synthesised according to methods c and b. The yield of the final step was 23.4 mg, 24.6%.
MS [M-CF3COO]⁺: 446

### Example 10

### (3R)-1-(3-Cyclohexylpropyl)-3-(2-hydroxy-2,2-dithien-2-ylacetoxy)-1-azoniabicyclo[2.2.2]octane trifluoroacetate

The title compound was synthesised according to methods c and b. The yield of the final step was 12.6 mg, 12.6%.
MS [M-CF3COO]⁺: 474

### Example 11

### (3R)-1-Allyl-3-(2-hydroxy-2,2-dithien-2-ylacetoxy)-1-azoniabicyclo[2.2.2]octane bromide

The title compound was synthesised according to methods c and a. The yield of the final step was 400 mg, 70.0%.
m.p.: 176°C
MS [M-Br]⁺ : 390
¹H-NMR (300 MHz, DMSO-d₆) δ ppm : 1.77 (m, 2 H), 1.92 (m, 2 H), 2.31 (m, 1 H), 3.15 (m, 1 H), 3.20-3.52 (m, 4 H), 3.81-4.01 (m, 3 H), 5.24 (m, 1 H), 5.53-5.68 (m, 2 H), 5.96 (m, 1 H), 7.02 (m, 2 H), 7.16 (m, 2 H), 7.48 (s, 1 H, OH), 7.53 (m, 2 H).

### Example 12

### (3R)-3-(2-Hydroxy-2,2-dithien-2-ylacetoxy)-1-(4-methylpent-3-enyl)-1-azoniabicyclo[2.2.2]octane bromide

The title compound was synthesised according to methods c and a. The yield of the final step was 260 mg, 36.0%.
m.p.: 199°C
MS [M-Br]⁺: 432
¹H-NMR (300 MHz, CDCl₃) δ ppm: 1.58 (s, 3 H), 1.64 (m, 3 H), 1.87 (m, 2 H), 2.03 (m, 2 H), 2.26-2.47 (m, 3 H), 3.22-3.45 (m, 2 H), 3.45-3.82 (m, 5 H), 4.23 (m, 1 H), 4.92 (m, 1 H), 5.27 (m, 1 H), 6.07 (s, 1 H, OH), 6.93 (m, 2 H), 7.15 (m, 1 H), 7.24 (m, 3 H).

### Example 13

### (3R)-1-(3,7-Dimethylocta-(E)-2,6-dienyl)-3-(2-hydroxy-2,2-dithien-2-ylacetoxy)-1-azoniabicyclo[2.2.2]octane trifluoroacetate

The title compound was synthesised according to methods c and b. The yield of the final step was 14.5 mg, 14.2%.
MS [M-CF3COO]⁺ : 486

### Example 14

### (3R)-3-(2-Hydroxy-2,2-dithien-2-ylacetoxy)-1-(2-hydroxyethyl)-1-azoniabicyclo[2.2.2]octane trifluoroacetate

The title compound was synthesised according to methods c and b. The yield of the final step was 16.6 mg, 19.3%.
MS [M-CF3COO]⁺ : 394

### Example 15

### (3R)-3-(2-Hydroxy-2,2-dithien-2-ylacetoxy)-1-(3-hydroxypropyl)-1-azoniabicyclo[2.2.2]octane trifluoroacetate

The title compound was synthesised according to methods c and b. The yield of the final step was 16.0 mg, 18.0%.
MS [M-CF3COO]⁺ : 408

### Example 16

### (3R)-1-(4-Hydroxybutyl)-3-(2-hydroxy-2,2-dithien-2-ylacetoxy)-1-azoniabicyclo[2.2.2]octane trifluoroacetate

The title compound was synthesised according to methods c and b. The yield of the final step was 6.5 mg, 7.1%.
MS [M-CF3COO]⁺ : 422

### Example 17

### (3R)-1-(2-Ethoxyethyl)-3-(2-hydroxy-2,2-dithien-2-ylacetoxy)-1-azoniabicyclo[2.2.2]octane bromide

The title compound was synthesised according to methods c and a. The yield of the final step was 220 mg, 31.0%.
m.p.: 155°C
MS [M-Br]⁺ : 422
¹H-NMR (300 MHz, CDCl₃) δ ppm: 1.15 (t, 3 H), 1.88 (m, 2 H), 2.03 (m, 2 H), 2.46 (m, 1 H), 3.49 (q, 2 H), 3.54-3.96 (m, 8 H), 4.06 (m, 1 H), 4.31 (m, 1 H), 5.27 (m, 1 H), 5.73 (s, 1 H, OH), 6.97 (m, 2 H), 7.20 (m, 1 H), 7.22-7.33 (m, 3 H).

### Example 18

### (3R)-3-(2-Hydroxy-2,2-dithien-2-ylacetoxy)-1-[2-(2-hydroxyethoxy)ethyl]-1-azoniabicyclo[2.2.2]octane chloride

The title compound was synthesised according to methods c and a. The yield of the final step was 580 mg, 14.0%.
m.p.: 156°C
MS [M-Cl]⁺: 438
¹H-NMR (300 MHz, DMSO-d₆) δ ppm : 1.72 (m, 2 H), 1.92 (m, 2 H), 2.27 (m, 1 H), 3.10-3.70 (m, 11 H), 3.79 (m, 2 H), 3.96 (m, 1 H), 4.72 (m, 1 H), 5.21 (m, 1 H), 6.97 (m, 2 H), 7.15 (m, 2 H), 7.51 (m, 3 H).

### Example 19

### (3R)-3-(2-Hydroxy-2,2-dithien-2-ylacetoxy)-1-[2-(2-methoxyethoxy)ethyl]-1-azoniabicyclo[2.2.2]octane bromide

The title compound was synthesised according to methods c and a. The yield of the final step was 260 mg, 35.0%.
MS [M-Br]⁺ : 452
¹H-NMR (300 MHz, CDCl₃) δ ppm: 1.87 (m, 2 H), 2.04 (m, 2 H), 2.44 (m, 1 H), 3.32 (s, 3 H), 3.48 (m, 2 H), 3.54-3.96 (m, 10 H), 3.98-4.08 (m, 1 H), 4.30 (m, 1 H), 5.26 (m, 1 H), 6.0 (b.s., 1 H, OH), 6.97 (m, 2 H), 7.20 (m, 1 H), 7.23-7.32 (m, 3 H).

### Example 20

### (3R)-3-(2-Hydroxy-2,2-dithien-2-ylacetoxy)-1-oxiranylmethyl-1-azoniabicyclo[2.2.2]octane bromide

The title compound was synthesised according to methods c and a. The yield of the final step was 240 mg, 35.0%.
MS [M-Br]⁺: 406

### Example 21

### (3R)-1-(2-[1,3]Dioxolan-2-ylethyl)-3-(2-hydroxy-2,2-dithien-2-ylacetoxy)-1-azoniabicyclo[2.2.2]octane trifluoroacetate

The title compound was synthesised according to methods c and b. The yield of the final step was 15.7 mg, 16.4%.
MS [M-CF3COO]⁺: 450

### Example 22

### (3R)-3-(2-Hydroxy-2,2-dithien-2-ylacetoxy)-1-{2-[2-(2-hydroxyethoxy)-ethoxy]ethyl}-1-azoniabicyclo[2.2.2]octane trifluoroacetate

The title compound was synthesised according to methods c and b. The yield of the final step was 7.9 mg, 7.8%.
MS [M-CF3COO]⁺: 482

### Example 23

### (3R)-1-(3-[1,3]Dioxolan-2-ylpropyl)-3-(2-hydroxy-2,2-dithien-2-ylacetoxy)-1-azoniabicyclo[2.2.2]octane trifluoroacetate

The title compound was synthesised according to methods c and b. The yield of the final step was 17.1 mg, 17.4%.
MS [M-CF3COO]⁺: 464

### Example 24

### (3R)-1-(3-Ethoxycarbonylpropyl)-3-(2-hydroxy-2,2-dithien-2-yl-acetoxy)-1-azoniabicyclo[2.2.2]octane trifluoroacetate

The title compound was synthesised according to methods c and b. The yield of the final step was 15.1 mg, 15.4%.
MS [M-CF3COO]⁺: 464

### Example 25

### (3R)-1-(4-Ethoxycarbonylbutyl)-3-(2-hydroxy-2,2-dithien-2-ylacetoxy)-1-azoniabicyclo[2.2.2]octane trifluoroacetate

The title compound was synthesised according to methods c and b. The yield of the final step was 15.0 mg, 14.9%.
MS [M-CF3COO]⁺: 478

### Example 26

### (3R)-1-(4-Acetoxybutyl)-3-(2-hydroxy-2,2-dithien-2-ylacetoxy)-1-azoniabicyclo[2.2.2]octane trifluoroacetate

The title compound was synthesised according to methods c and b. The yield of the final step was 10.7 mg, 10.9%.
MS [M-CF3C00]⁺ : 464

### Example 27

### (3R)-1-(3-Cyanopropyl)-3-(2-hydroxy-2,2-dithien-2-ylacetoxy)-1-azoniabicyclo[2.2.2]octane trifluoroacetate

The title compound was synthesised according to methods c and b. The yield of the final step was 14.0 mg, 15.5%.
MS [M-CF3COO]⁺: 417

### Example 28

### (3R)-1-(4-Cyanobutyl)-3-(2-hydroxy-2,2-dithien-2-ylacetoxy)-1-azoniabicyclo[2.2.2]octane trifluoroacetate

The title compound was synthesised according to methods c and b. The yield of the final step was 16.2 mg, 17.5%.
MS [M-CF3COO]⁺: 431

### Example 29

### (3R)-1-(6-Cyanohexyl)-3-(2-hydroxy-2,2-dithien-2-ylacetoxy)-1-azoniabicyclo[2.2.2]octane trifluoroacetate

The title compound was synthesised according to methods c and b. The yield of the final step was 19.1 mg, 19.6%.
MS [M-CF3COO]⁺: 459

### Example 30

### (3R)-3-(2-Hydroxy-2,2-dithien-2-ylacetoxy)-1-(4,4,4-trifluorobutyl)-1-azoniabicyclo[2.2.2]octane trifluoroacetate

The title compound was synthesised according to methods c and b. The yield of the final step was 18.4 mg, 18.8%.
MS [M-CF3COO]⁺: 460

### Example 31

### (3R)-1-Allyl-3-(2-hydroxy-2-phenyl-2-thien-2-ylacetoxy)-1-azoniabicyclo[2.2.2]octane bromide

The title compound was synthesised as a mixture of diastereomers according to methods f and a. The yield of the final step was 350 mg, 50.0%.
m.p.: 170°C
MS [M-Br]⁺ : 384
¹H-NMR (300 MHz, DMSO-d₆) δ ppm : (mixture of diastereomers) 1.46-1.79 (m, 2 H), 1.81-2.02 (m, 2 H), 2.28 (m, 1 H), 3.07 (m, 1 H), 3.17-3.46 (m, 4 H), 3.80-4.0 (m, 3 H), 5.23 (m, 1 H), 5.53-5.67 (m, 2 H), 5.96 (m, 1 H), 7.03 (m, 1 H), 7.12 (m, 1 H), 7.20 (s, 1 H, OH), 7.29-7.42 (m, 3 H), 7.42-7.49 (m, 2 H), 7.53 (m, 1 H).

### Example 32

### (3R)-1-Allyl-3-(2-fur-2-yl-2-hydroxy-2-phenylacetoxy)-1-azonlabicyclo[2.2.2]octane bromide

The title compound was synthesised as a mixture of diastereomers according to methods c and a. The yield of the final step was 120 mg, 88.8%.
MS [M-Br]⁺: 368
¹H-NMR (300 MHz, DMSO-d₆) δ ppm : (mixture of diastereomers) 1.45-1.80 (m, 2 H), 1.80-2.03 (m, 2 H), 2.26 (m, 1 H), 2.95-3.24 (m, 2 H), 3.24-3.47 (m, 3 H), 3.75-3.98 (m, 3 H), 5.20 (m, 1 H), 5.52-5.67 (m, 2 H), 5.96 (m, 1 H), 6.28 (dd, 1 H), 6.46 (m, 1 H), 6.95 (d, 1 H, OH), 7.31-7.45 (m, 3 H), 7.48 (m, 2 H), 7.68 (m, 1 H).

### Example 33

### (3R)-1-Allyl-3-(2-fur-2-yl-2-hydroxy-2-thien-2-ylacetoxy)-1-azoniabicyclo[2.2.2]octane bromide

The title compound was synthesised as a mixture of diastereomers according to methods c and a. The yield of the final step was 170 mg, 62.9%.
MS [M-Br]⁺: 374
¹H-NMR (300 MHz, DMSO-d₆) δ ppm : (mixture of diastereomers) 1.58-1.83 (m, 2 H), 1.83-2.06 (m, 2 H), 2.28 (m, 1 H), 3.06 (m, 1 H), 3.18 (m, 1 H), 3.25-3.54 (m, 3 H), 3.78-4.02 (m, 3 H), 5.22 (m, 1 H), 5.52-5.68 (m, 2 H), 5.97 (m, 1 H), 6.33 (dd, 1 H), 6.46 (m, 1 H), 7.04 (m, 1 H), 7.15 (m, 1 H), 7.29 (d, 1 H), 7.55 (m, 1 H), 7.69 (m, 1 H).

### Example 34

### (3R)-1-Allyl-3-(9-methyl-9H-fluorene-9-carbonyloxy)-1-azoniabicyclo[2.2.2]octane bromide

The title compound was synthesised according to methods c and a. The yield of the final step was 180 mg, 88.2%.
m.p.: 75.2-76.8°C
MS [M-Br]⁺ : 374
¹H-NMR (300 MHz, DMSO-d₆) δ ppm : 1.54 (m, 1 H), 1.69 (m, 1 H), 1.76 (s, 3 H), 1.87 (m, 2 H), 2.11 (m, 1 H), 3.00 (m, 1 H), 3.16-3.47 (m, 4 H), 3.79 (m, 1 H), 3.91 (m, 2 H), 4.99 (m, 1 H), 5.52-5.66 (m, 2 H), 5.93 (m, 1 H), 7.30-7.50 (m, 4 H), 7.64 (d, 1 H), 7.72 (d, 1 H), 7.90 (d, 2 H).

### Example 35

### (3R)-1-Allyl-3-(9-hydroxy-9H-fluorene-9-carbonyloxy)-1-azoniabicyclo[2.2.2]octane bromide

The title compound was synthesised according to methods c, and a from Intermediate 1-3. The yield of the final step was 550 mg, 80.3%.
m.p.: 260°C
MS [M-Br]⁺ : 376
¹H-NMR (300 MHz, DMSO-d₆) δ ppm : 1.40 (m, 1 H), 1.64 (m, 1 H), 1.85 (m, 2 H), 2.08 (m, 1 H), 2.77 (m, 1 H), 3.03 (m, 1 H), 3.17-3.41 (m, 3 H), 3.70-3.94 (m, 3 H), 5.03 (m, 1 H), 5.50-5.68 (m, 2 H), 5.90 (m, 1 H), 6.85 (s, 1 H, OH), 7.35 (m, 2 H), 7.46 (m, 2 H), 7.60 (m, 2 H), 7.83 (d, 2 H).

### Example 36

### (3R)-3-(9-Hydroxy-9H-fluorene-9-carbonyloxy)-1-(4-methylpent-3-enyl)-1-azoniabicyclo[2.2.2]octane bromide

The title compound was synthesised according to methods c, and a from Intermediate 1-3. The yield of the final step was 490 mg, 65.5%.
m.p.: 192.4-193.1 °C
MS [M-Br]⁺: 418
¹H-NMR (300 MHz, DMSO-d₆) δ ppm: 1.40 (m, 1 H), 1.55-1.72 (m, 1 H), 1.61 (s, 3 H), 1.66 (s, 3 H), 1.84 (m, 2 H), 2.04 (m, 1 H), 2.26 (m, 2 H), 2.76 (m, 1 H), 2.98-3.16 (m, 3 H), 3.18-3.45 (m, 3 H), 3.79 (m, 1 H), 4.95 (m, 1 H), 5.01 (m, 1 H), 6.82 (s, 1 H, OH), 7.33 (m, 2 H), 7.44 (m, 2 H), 7.59 (m, 2 H), 7.82 (d, 2 H).

### Example 37

### (3R)-1-Heptyl-3-(9-hydroxy-9H-fluorene-9-carbonyloxy)-1-azoniabicyclo[2.2.2]octane bromide

The title compound was synthesised according to methods c, and a from Intermediate 1-3. The yield of the final step was 330 mg, 85.5%.
m.p.: 214.9-216.6°C
MS [M-Br]⁺ : 434
¹H-NMR (300 MHz, DMSO-d₆) δ ppm: 0.88 (t, 3 H), 1.12-1.70 (m, 12 H), 1.70-1.93 (m, 2 H), 2.04 (m, 1 H), 2.78 (m, 1 H), 2.96-3.18 (m, 3 H), 3.18-3.48 (m, 3 H), 3.77 (m, 1 H), 5.01 (m, 1 H), 6.84 (s, 1 H, OH), 7.34 (m, 2 H), 7.45 (m, 2 H), 7.61 (m, 2 H), 7.84 (d, 2 H).

### Example 38

### (3R)-3-(9-Hydroxy-9H-fluorene-9-carbonyloxy)-1-oxiranylmethyl-1-azoniabicyclo[2.2.2]octane bromide

The title compound was synthesised according to methods c, and a from Intermediate I-3. The yield of the final step was 270 mg, 38.0%.
MS [M-Br]⁺: 392

### Example 39

### (3R)-3-(9-Hydroxy-9H-fluorene-9-carbonyloxy)-1-[2-(2-methoxyethoxy)ethyl]-1-azoniabicyclo[2.2.2]octane bromide

The title compound was synthesised according to methods c, and a from Intermediate 1-3. The yield of the final step was 610 mg, 78.2%.
m. p.: 194°C
MS [M-Br]⁺ : 438
¹H-NMR (300 MHz, DMSO-d₆) δ ppm: 1.44 (m, 1 H), 1.64 (m, 1 H), 1.85 (m, 2 H), 2.09 (m, 1 H), 2.93 (m, 1 H), 3.18-3.28 (m, 1 H), 3.26 (s, 3 H), 3.30-3.60 (m, 9 H), 3.73 (m, 2 H), 3.88 (m, 1 H), 5.00 (m, 1 H), 6.83 (s, 1 H, OH), 7.35 (m, 2 H), 7.45 (m, 2 H), 7.61 (m, 2 H), 7.83 (d, 2 H).

### Example 40

### (3R)-1-(2-[1,3]Dioxolan-2-ylethyl)-3-(9-hydroxy-9H-fluorene-9-carbonyloxy)-1-azoniabicyclo[2.2.2]octane bromide

The title compound was synthesised according to methods c, and a from Intermediate 1-3. The yield of the final step was 660 mg, 85.7%.
m.p.: 62°C
MS [M-Br]⁺ : 436
¹H-NMR (300 MHz, DMSO-d₆) δ ppm: 1.43 (m, 1 H), 1.64 (m, 1 H), 1.71-2.15 (m, 5 H), 2.86 (m, 1 H), 3.0-3.64 (m, 8 H), 3.74-3.97 (m, 3 H), 4.89 (m, 1 H), 5.00 (s, 1 H), 7.34 (m, 2 H), 7.45 (m, 2 H), 7.63 (m, 2 H), 7.82 (d, 2 H).

### Example 41

### (3R)-1-Allyl-3-(9H-xanthene-9-carbonyloxy)-1-azoniabicyclo[2.2.2]octane bromide

The title compound was synthesised according to methods d and a. The yield of the final step was 170 mg, 51.5 %.
m.p.: 57°C
MS [M-Br]⁺ : 376
¹H-NMR (300 MHz, DMSO-d₆) δ ppm: 1.63-2.00 (m, 4 H), 2.17 (m, 1 H), 3.06-3.56 (m, 5 H), 3.77 (m, 1 H), 3.92 (m, 2 H), 5.03 (m, 1 H), 5.30 (s, 1 H), 5.50-5.70 (m, 2 H), 5.94 (m, 1 H), 7.20 (m, 4 H), 7.38 (m, 2 H), 7.50 (m, 2 H).

### Example 42

### (3R)-1-(4-Methylpent-3-enyl)-3-(9H-xanthene-9-carbonyloxy)-1-azoniabicyclo[2.2.2]octane bromide

The title compound was synthesised according to methods d and a. The yield of the final step was 270 mg, 72.9%.
m.p.: 225°C
MS [M-Br]⁺ : 418
¹H-NMR (300 MHz, DMSO-d₆) δ ppm: 1.60-2.0 (m, 4 H), 1.64 (s, 3 H), 1.69 (s, 3 H), 2.16 (m, 1 H), 2.32 (m, 2 H), 3.10-3.46 (m, 6 H), 3.53 (m, 1 H), 3.85 (m, 1 H), 4.93-5.08 (m, 2 H), 5.32 (s, 1 H), 7.19 (m, 4H), 7.38 (m, 2 H), 7.52 (m, 2 H).

### Example 43

### (3R)-1-Allyl-3-(9-methyl-9H-xanthene-9-carbonyloxy)-1-azoniabicyclo[2.2.2]octane bromide

The title compound was synthesised according to methods c and a. The yield of the final step was 1.04 g, 73.8%.
m.p.: 64.3-67.8°C
MS [M-Br]⁺ : 390
¹H-NMR (300 MHz, DMSO-d₆) δ ppm: 1.35 (m, 1 H), 1.65 (m, 1 H), 1.73-1.91 (m, 2 H), 1.89 (s, 3 H), 2.11 (m, 1 H), 2.75 (m, 1 H), 3.00 (m, 1 H), 3.15-3.45 (m, 3 H), 3.70-3.90 (m, 3 H), 5.03 (m, 1 H), 5.50-5.65 (m, 2 H), 5.86 (m, 1 H), 7.12-7.19 (m, 4 H), 7.35 (m, 2 H), 7.42 (m, 2 H).

### Example 44

### (3R)-1-Allyl-3-(9-hydroxy-9H-xanthene-9-carbonyloxy)-1-azoniabicyclo[2.2.2]octane bromide

The title compound was synthesised according to methods c and a. The yield of the final step was 270 mg, 67.5%.
m.p.: 232°C
MS [M-Br]⁺ : 392
¹H-NMR (300 MHz, DMSO-d₆) δ ppm: 1.29 (m, 1 H), 1.62 (m, 1 H), 1.70-1.94 (m, 2 H), 2.06 (m, 1 H), 2.62 (m, 1 H), 2.79 (m, 1 H), 3.13-3.42 (m, 3 H), 3.64-3.87 (m, 3 H), 5.02 (m, 1 H), 5.47-5.65 (m, 2 H), 5.81 (m, 1 H), 7.18 (s, 1 H, OH), 7.25 (m, 4 H), 7.44 (m, 2 H), 7.65 (m, 2 H).

### Example 45

### (3R)-1-(3-Hydroxypropyl)-3-(9-hydroxy-9H-xanthene-9-carbonyloxy)-1-azoniabicyclo[2.2.2]octane bromide

The title compound was synthesised according to methods c and a. The yield of the final step was 315 mg, 90.5%.
m.p.: 87.6-89.1°C
MS [M-Br]⁺ : 410
¹H-NMR (300 MHz, DMSO-d₆) δ ppm: 1.28 (m, 1 H), 1.50-1.95 (m, 5 H), 2.05 (m, 1 H), 2.63 (m, 1 H), 2.83 (m, 1 H), 3.10-3.50 (m, 7 H), 3.73 (m, 1 H), 4.76 (t, 1 H, OH), 5.0 (m, 1 H), 7.18 (s, 1 H, OH), 7.18-7.30 (m, 4 H), 7.40-7.50 (m, 2 H), 7.60-7.70 (m, 2 H).

### Example 46

### (3R)-1-Allyl-3-(10,11-dihydro-5H-dibenzo[a,d]cycloheptene-5-carbonyloxy)-1-azoniabicyclo[2.2.2]octane bromide

The title compound was synthesised according to methods d, and a from Intermediate I-4. The yield of the final step was 260 mg, 95.6%.
m.p.: 219.5-220.3°C
MS [M-Br]⁺ : 388
¹H- NMR (300 MHz, CDCl₃) δ ppm: 1.52 (m, 1 H), 1.71 (m, 1 H), 1.93 (m, 2 H), 2.20 (m, 1 H), 2.79-2.94 (m, 2 H), 3.02 (m, 1 H), 3.11-3.31 (m, 2 H), 3.40-3.52 (m, 1 H), 3.52-3.75 (m, 3 H), 4.06-4.39 (m, 3 H), 5.10 (m, 1 H), 5.21 (s, 1 H), 5.57-5.87 (m, 3 H), 7.16 (m, 6 H), 7.39 (m, 2 H).

### Example 47

### (3R)-1-(4-Methylpent-3-enyl)-3-(10,11-dihydro-5H-dibenzo[a,d]cycloheptene-6-carbonyloxy)-1-azoniabicyclo[2.2.2]octane bromide

The title compound was synthesised according to methods d, and a from Intermediate I-4. The yield of the final step was 290 mg, 97.9 %.
m.p.: 216°C
MS [M-Br]⁺ : 430
¹H- NMR (300 MHz, CDCl₃) δ ppm: 1.53 (m, 1 H), 1.62 (s, 3 H), 1.67 (s, 3 H), 1.81 (m, 1 H), 1.98 (m, 2 H), 2.18-2.35 (m, 3 H), 2.75-2.98 (m, 3 H), 3.11-3.46 (m, 5 H), 3.54-3.76 (m, 3 H), 4.20 (m, 1 H), 4.95 (m, 1 H), 5.14 (s, 1 H), 5.10-5.20 (m, 1 H), 7.19 (m, 6 H), 7.36 (m, 2 H).

### Example 48

### (3R)-1-Allyl-3-[(2*)-2-hydroxy-2,3-diphenylpropionyloxy]-1-azoniabicyclo[2.2.2]octane bromide (diastereomer 1)

The title compound was synthesised according to methods f, and a from Intermediate 1-6a. The yield of the final step was 250 mg, 75.7%.
m.p.: 180°C
MS [M-Br]⁺ : 392
¹H-NMR (300 MHz, DMSO-d₆) δ ppm: 1.72 (m, 2 H), 1.92 (m, 2 H), 2.21 (m, 1 H), 3.02 (m, 1 H), 3.11 (m, 1 H), 3.18-3.52 (m, 5 H), 3.74 (m, 1 H), 3.84 (m, 2 H), 5.04 (m, 1 H), 5.49-5.64 (m, 2 H), 5.90 (m, 1 H), 6.15 (s, 1 H, OH), 7.14 (m, 5 H), 7.23-7.40 (m, 3 H), 7.53 (m, 2 H). (*) Configuration not assigned

### Example 49

### (3R)-3-[(2*)-2-Hydroxy-2,3-diphenylpropionyloxy)]-1-(4-methylpent-3-enyl)-1-azoniabicyclo[2.2.2]octane bromide (diastereomer 1)

The title compound was synthesised according to methods f, and a from Intermediate I-6a. The yield of the final step was 280 mg, 77.7%.
m.p.: 224°C
MS [M-Br]⁺ : 434
¹H-NMR (300 MHz, DMSO-d₆) δ ppm: 1.61 (s, 3 H), 1.67 (s, 3 H), 1.63-1.75 (m, 2 H), 1.75-2.00 (m, 2 H), 2.21 (m, 1 H), 2.28 (m, 2 H), 2.99-3.20 (m, 4 H), 3.20-3.50 (m, 5 H), 3.77 (m, 1 H), 4.97 (m, 1 H), 5.04 (m, 1 H), 6.14 (s, 1 H, OH), 7.14 (m, 5 H), 7.23-7.39 (m, 3 H), 7.54 (m, 2 H).
(*) Configuration not assigned

### Example 50

### (3R)-1-Allyl-3-(2-hydroxy-2-thien-2-ylpent-4-enoyloxy)-1-azoniabicyclo[2.2.2]octane bromide

The title compound was synthesised as a mixture of diastereomers according to methods c and a. The yield of the final step was 210 mg, 61.8 %.
m.p.: 62.6-63.9°C
MS [M-Br]⁺ : 348
¹H-NMR (300 MHz, DMSO-d₆) δ ppm: (mixture of diastereomers) 1.68-2.05 (m, 4 H), 2.26 (m, 1 H), 2.76 (m, 1 H), 2.95 (m, 1 H), 3.08-3.53 (m, 5 H), 3.74-4.00 (m, 3 H), 5.02-5.21 (m, 3 H), 5.51-5.67 (m, 2 H), 5.78 (m, 1 H), 5.97 (m, 1 H), 6.49 (d, 1 H, OH), 7.01 (m, 1 H), 7.15 (m, 1 H), 7.48 (m, 1 H)

### Example 51

### (3R)-1-(4-Methylpent-3-enyl)-3-(2-Hydroxy-2-thien-2-ylpent-4-enoyloxy)-1-azoniabicyclo[2.2.2]octane bromide

The title compound was synthesised as a mixture of diastereomers according to methods c and a. The yield of the final step was 290 mg, 78.4 %.
m.p.: 56.2-57.9°C
MS [M-Br]⁺ : 390
¹H-NMR (300 MHz, DMSO-d₆) δ ppm: (mixture of diastereomers) 1.52-2.06 (m, 4 H), 1.63 (d, 3 H), 1.69 (s, 3 H), 2.16-2.43 (m, 3 H), 2.78 (dd, 1 H), 2.96 (m, 1 H), 3.07-3.59 (m, 7 H), 3.88 (m, 1 H), 4.92-5.23 (m, 4 H), 5.78 (m, 1 H), 6.48 (d, 1 H, OH), 7.01 (m, 1 H), 7.15 (m, 1 H), 7.46 (m, 1 H).

### Example 52

### (3R)-1-Allyl-3-[(2S)-2-cyclopentyl-2-hydroxy-2-thien-2-ylacetoxy]-1-azoniabicyclo[2.2.2]octane bromide

The title compound was synthesised according to methods f and a. The yield of the final step was 180 mg, 78.3 %.
m.p.: 68.2-70.1°C
MS [M-Br]⁺ : 376
¹H-NMR (300 MHz, DMSO-d₆) δ ppm: 1.26-1.66 (m, 8 H), 1.78-2.08 (m, 4 H), 2.30 (m, 1 H), 2.81 (m, 1 H), 3.14 (m, 1 H), 3.20-3.58 (m, 4 H), 3.78-4.03 (m, 3 H), 5.15 (m, 1 H), 5.52-5.68 (m, 2 H), 5.97 (m, 1 H), 6.18 (s, 1 H, OH), 7.01 (m, 1 H), 7.16 (d, 1 H), 7.45 (d, 1 H).

### Example 53

### (3R)-1-(4-Methylpent-3-enyl)-3-[(2S)-2-cyclopentyl-2-hydroxy-2-thien-2-ylacetoxy)-1-azoniabicyclo[2.2.2]octane bromide

The title compound was synthesised according to methods f and a. The yield of the final step was 290 mg, 71.7%.
m.p.: 84°C
MS [M-Br]⁺ : 418
¹H-NMR (300 MHz, DMSO-d₆) δ ppm: 1.19-1.76 (m, 8 H), 1.63 (s, 3 H), 1.68 (s, 3 H), 1.77-2.05 (m, 4 H), 2.20-2.42 (m, 3 H), 2.81 (m, 1 H), 3.11-3.24 (m, 3 H), 3.24-3.61 (m, 4 H), 3.89 (m, 1 H), 5.01 (m, 1 H), 5.14 (m, 1 H), 6.19 (s, 1 H, OH), 7.00 (m, 1 H), 7.16 (d, 1 H), 7.44 (d, 1 H).

### Example 54

### (3R)-1-Allyl-3-[(2R)-2-cyclopentyl-2-hydroxy-2-thien-2-ylacetoxy)-1-azoniabicyclo[2.2.2]octane bromide

The title compound was synthesised according to methods c and a. The yield of the final step was 230 mg, 85.2 %.
m.p.: 65.3-66.0°C
MS [M-Br]⁺ : 376
¹H-NMR (300 MHz, DMSO-d₆) δ ppm: (mixture of diastereomers (3R,2R):(3R,2S) 72:28) 1.30-1.66 (m, 8 H), 1.67-2.05 (m, 4 H), 2.23 and 2.30 (m, 1 H), 2.83 (m, 1 H), 3.06-3.55 (m, 5 H), 3.74-4.03 (m, 3 H), 5.14 (m, 1 H), 5.53-5.68 (m, 2 H), 5.99 (m, 1 H), 6.18 and 6.19 (s, 1 H, OH), 7.00 (m, 1 H), 7.16 (m, 1 H), 7.44 (m, 1 H).

### Example 55

### (3R)-3-[(2S)-2-Cyclopentyl-2-hydroxy-2-thien-2-ylacetoxy]-1-(2-hydroxyethyl)-1-azoniabicyclo[2.2.2]octane bromide

The title compound was synthesised according methods f and a. The crude mixture was purified by reverse chromatography eluting the product with water. The yield of the final step was 148 mg, 66%.
MS [M-Br]⁺: 380
¹H-NMR (300 MHz, DMSO-d₆) δ ppm: 1.25-1.99 (m, 12H), 2.29 (m, 1H), 2.75-2.85 (m, 1 H), 3.30-3.60 (m, 7H), 5.13 (m, 1 H), 5.29-5.32 (t, 1H), 6.21 (s, 1H), 6.98-7.01 (dd, 1H), 7.13-7.15 (dd, 1 H), 7.42-7.44 (dd, 1 H)

### Example 56

### (3R)-3-[(2S)-2-Cyclopentyl-2-hydroxy-2-thien-2-ylacetoxy]-1-(3-hydroxypropyl)-1-azoniabicyclo[2.2.2]octane bromide

The title compound was synthesised according methods f and a. The crude mixture was purified by reverse chromatography eluting the product with water. The yield of the final step was 145 mg, 53%.
MS [M-Br]⁺: 394

### Example 57

### (3R)-3-[(2S)-2-Cyclopentyl-2-hydroxy-2-thien-2-ylacetoxy]-1-(4-hydroxybutyl)-1-azoniabicyclo[2.2.2]octane bromide

The title compound was synthesised according methods f and a. The crude mixture was purified by reverse chromatography eluting the product with water. The yield of the final step was 40 mg, 20%.
MS [M-Br]⁺: 408
¹H-NMR (300 MHz, DMSO-d₆) δ ppm: 1.25-1.93 (m, 18H), 2.29 (m, 1H), 2.81 (m, 1 H), 3.08-3.48 (m, 5H), 3.75-3.90 (m, 1H), 4.61-4.64 (tm 1H), 5.10-5.20 (m, 1H), 6.23 (s, 1H), 6.99-7.02 (t, 1H), 7.14-7.16 (m, 1 H), 7.43-7.45 (d, 1 H)

### Example 58

### (3R)-3-[(2S)-2-Cyclopentyl-2-hydroxy-2-thien-2-ylacetoxy]-1-[2-(2-hydroxyethoxy)ethyl]-1-azoniabicyclo[2.2.2]octane bromide

The title compound was synthesised according methods f and a. The crude mixture was purified by reverse chromatography eluting the product with water. The yield of the final step was 110 mg, 37%.
MS [M-Br]⁺: 424
¹H-NMR (300 MHz, DMSO-d₆) δ ppm: 1.10-1.53 (m, 14H), 1.82-2.04 (m, 3H), 2.17-2.37 (m, 1H), 2.76-2.87 (m, 1H), 3.23-3.51 (m, 5H), 3.79-3.95 (m, 2H), 4.67-4.71 (t, 1 H), 5.09-5.19 (m, 1H), 6.20 (s, 1H), 6.99-7.01 (dd, 1H), 7.14-7.16 (m, 1H), 7.42-7.44 (dd, 1H)

### Example 59

### (3R)-3-[(2S)-2-Cyclopentyl-2-hydroxy-2-thien-2-ylacetoxy]-1-(6-hydroxyhexyl)-1-azoniabicyclo[2.2.2]octane bromide

The title compound was synthesised according methods f and a. The crude mixture was purified by reverse chromatography eluting the product with water. The yield of the final step was 90
mg, 35%.
MS [M-Br]⁺: 436
¹H-NMR (300 MHz, DMSO-d₆) δ ppm: 1.20-2.00 (m, 20H), 2.28 (m, 1H), 2.81 (m, 1H), 3.10-3.50 (m, 9H), 3.81 (m, 1 H), 4.38-4.41 (t, 1H), 5.12 (m, 1 H), 6.20 (s, 1H), 6.69-7.02 (t, 1H), 7.14-7.15 (m, 1H), 7.43-7.46 (m, 1 H)

The following examples illustrate pharmaceutical compositions according to the present invention and procedures for their preparation.

### Example 60

### Preparation of a pharmaceutical composition: tablets

| Formulation: | |
|---|---|
| Compound of the present invention | 5.0 mg |
| Lactose | 113.6 mg |
| Microcrystalline cellulose | 28.4 mg |
| Light silicic anhydride | 1.5 mg |
| Magnesium stearate | 1.5 mg |

Using a mixer machine, 15 g of the compound of the present invention was mixed with 340.8 g of lactose and 85.2 g of microcrystalline cellulose. The mixture was subjected to compression moulding using a roller compactor to give a flake-like compressed material. The flake-like compressed material was pulverized using a hammer mill, and the pulverized material was screened through a 20 mesh screen. A 4.5 g portion of light silicic anhydride and 4.5 g of magnesium stearate were added to the screened material and mixed. The mixer product was subjected to a tablets making machine equipped with a die/punch system of 7.5 mm in diameter, thereby obtaining 3,000 tablets each having 150 mg in weight.

### Example 61

### Preparation of a pharmaceutical composition: tablets coated

| Formulation: | |
|---|---|
| Compound of the present invention | 5.0 mg |
| Lactose | 95.2 mg |
| Corn starch | 40.8 mg |
| Polyvinylpyrrolidone K25 | 7.5 mg |
| Magnesium stearate | 1.5 mg |
| Hydroxypropylcellulose | 2.3 mg |
| Polyethylene glycol 6000 | 0.4 mg |
| Titanium dioxide | 1.1 mg |
| Purified talc | 0.7 mg |

Using a fluidized bed granulating machine, 15 g of the compound of the present invention was mixed with 285.6 g of lactose and 122.4 g of corn starch. Separately, 22.5 g of polyvinylpyrrolidone was dissolved in 127.5 g of water to prepare a binding solution. Using a fluidized bed granulating machine, the binding solution was sprayed on the above mixture to give granulates. A 4.5 g portion of magnesium stearate was added to the obtained granulates and mixed. The obtained mixture was subjected to a tablet making machine equipped with a die/punch biconcave system of 6.5 mm in diameter, thereby obtaining 3,000 tablets, each having 150 mg in weight.

Separately, a coating solution was prepared by suspending 6.9 g of hydroxypropylmethylcellulose 2910, 1.2 g of polyethylene glycol 6000, 3.3 g of titanium dioxide and 2.1 g of purified talc in 72.6 g of water. Using a High Coated, the 3,000 tablets prepared above were coated with the coating solution to give film-coated tablets, each having 154.5 mg in weight.

### Example 62

### Preparation of a pharmaceutical composition: liquid inhalant

| Formulation: | |
|---|---|
| Compound of the present invention | 400 µg |
| Physiological saline | 1 ml |

A 40 mg portion of the compound of the present invention was dissolved in 90 ml of physiological saline, and the solution was adjusted to a total volume of 100 ml with the same saline solution, dispensed in 1 ml portions into 1 ml capacity ampoule and then sterilized at 115° for 30 minutes to give liquid inhalant.

### Example 63

### Preparation of a pharmaceutical composition: powder inhalant

| Formulation: | |
|---|---|
| Compound of the present invention | 200 µg |
| Lactose | 4,000 µg |

A 20 g portion of the compound of the present invention was uniformly mixed with 400 g of lactose, and a 200 mg portion of the mixture was packed in a powder inhaler for exclusive use to produce a powder inhalant.

### Example 64

### Preparation of a pharmaceutical composition: inhalation aerosol.

| Formulation: | |
|---|---|
| Compound of the present invention | 200 µg |
| Dehydrated (Absolute) ethyl alcohol USP | 8,400 µg |
| 1,1,1,2-Tetrafluoroethane (HFC-134A) | 46,810 µg |

The active ingredient concentrate is prepared by dissolving 0.0480 g of the compound of the present invention in 2.0160 g of ethyl alcohol. The concentrate is added to an appropriate filling apparatus. The active ingredient concentrate is dispensed into aerosol container, the headspace of the container is purged with Nitrogen or HFC-134A vapor (purging ingredients should not contain more than 1 ppm oxygen) and is sealed with valve. 11.2344 g of HFC-134A propellant is then pressure filled into the sealed container

## Claims

1. A compound of formula (I): 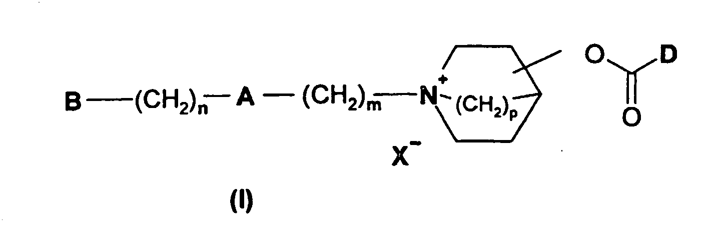 wherein B represents a hydrogen atom or a group selected from -R¹, -OR¹, hydroxy, -O(CO)R¹, cyano and an optionally substituted non-aromatic heterocyclyl containing one or more heteroatoms, wherein
R¹ is selected from the group consisting of hydrogen atoms, optionally substituted C₁₋₈ alkyl, optionally substituted C₂₋₈ alkenyl and optionally substituted C₃- C₈ cycloalkyl
n is an integer from 0 to 4;
A represents a group selected from -CH₂-, -CH=CR³-, -CR³=CH-, -CR³R⁴-, -O-, -CO-, -O-(CH₂)₂-O- wherein R³ and R⁴ each independently represent a hydrogen atom or a C₁₋₈ alkyl group;
m is an integer from 0 to 8;
p is an integer from 1 to 2
and the substitution in the azonia bicyclic ring may be in the 2, 3 or 4 position including all possible configurations of the asymmetric centers;
D is a group selected from: 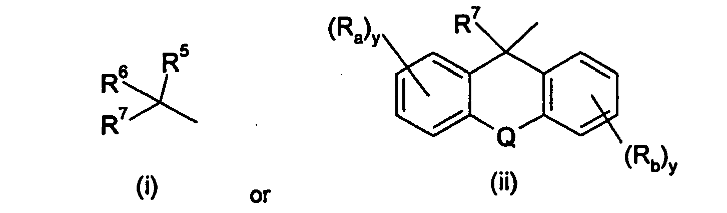 wherein R⁵ represents a group selected from phenyl, 2-thienyl, 3-thienyl, 2-furanyl, 3-furanyl which group may be optionally substituted by one or more substitutent Rₐ;
R⁶ represents a group selected from 2-thienyl, 3-thienyl, 2-furanyl, 3-furanyl, C₃- C₈ cycloalkyl, C₃- C₈ cycloalkenyl, C₁₋₈ alkyl, C₂₋₈ alkenyl, C₂₋₈ alkynyl, benzyl and phenylethyl which group may be optionally substituted by one or more substitutents R_{b};
R⁷ represents a hydrogen atom or a group selected from hydroxyl, hydroxymethyl and methyl;
Q represents a single bond or a group selected from -CH₂-, -CH₂CH₂-, -O-, -O-CH₂-, -S-, -S-CH₂-, and -CH=CH-;
Rₐ and R_{b} independently represent a group selected from halogen atoms, optionally substituted C₁₋₈ alkyl, optionally substituted C₁₋₈ alkoxy, hydroxy, trifluoromethyl, nitro, cyano, -COOR⁸, -NR⁸R⁹ wherein R⁸ and R⁹ independently represent a hydrogen atom or a C₁₋₈ alkyl group.
y is an integer from 0 to 3
X⁻ represents a pharmaceutically acceptable anion of a mono or polyvalent acid;
provided that the group B-(CH₂)ₙ-A-(CH₂)ₘ- is not a linear C₁₋₄ alkyl group and further provided that the compound is not one of:
1-Allyloxycarbonylmethyl-3-(2-hydroxy-2,2-di-thiophen-2-yl-acetoxy)-1-azonia-bicyclo-[2.2.2]octane; and
1-carboxymethyl-3-(2-hydroxy-2,2-di-thiophen-2-yl-acetoxy)-1-azonia-bicyclo-[2.2.2]octane.

2. A compound according to claim 1 wherein B is selected from the group consisting of hydrogen atoms, hydroxy groups, optionally substituted C₁₋₈ alkyl, optionally substituted C₂₋₈ alkenyl, optionally substituted C₃-C₈ cycloalkyl groups and non-aromatic heterocyclyl groups substituted at least with a hydroxy group..

3. A compound according to claim 1 wherein the azoniabicyclo group is substituted on the nitrogen atom with a group selected from allyl, 4-methylpent-3-enyl, isopropyl, cyclopropylmethyl, isobutyl, heptyl, cyclohexylmethyl, 3-cyclohexylpropyl, 3,7-dimethylocta-(E)-2,6-dienyl, 2-hydroxyethyl, 3-hydroxypropyl, 4-hydroxybutyl, 5-hydroxypentyl, 6-hydroxyhexyl, 2-ethoxyethyl, 2-(2-hydroxyethoxy)ethyl, 2-(2-methoxyethoxy)ethyl, oxiranylmethyl, 2-[1,3]dioxolan-2-ylethyl, 2-[2-(2-hydroxyethoxy)-ethoxy]ethyl, 3-[1,3]dioxolan-2-ylpropyl, 2-ethoxycarbonylethyl, 3-ethoxycarbonylpropyl, 4-ethoxycarbonylbutyl, 4-acetoxybutyl, 2-cyanoethyl, 3-cyanopropyl, 4-cyanobutyl, 6-cyanohexyl, 4,4,4-trifluorobutyl, 3-(4-hydroxypiperidin-1-yl)propyl and 4-(4-hydroxypiperidin-1-yl)butyl.

4. A compound according to claim 3 wherein the azoniabicyclo group is substituted on the nitrogen atom with a group selected from allyl, 4-methylpent-3-enyl, isopropyl, cyclopropylmethyl, isobutyl, heptyl, 2-hydroxyethyl, 3-hydroxypropyl, 4-hydroxybutyl, 5-hydroxypentyl, 6-hydroxyhexyl, 2-(2-methoxyethoxy)ethyl, 2-(2-hydroxyethoxy)ethyl, 4-ethoxycarbonylbutyl, 4-acetoxybutyl, 3-cyanopropyl and 4-cyanobutyl.

5. A compound according to any preceding claim wherein p is 2.

6. A compound according to any preceding claim wherein the substitution in the azonia bicyclic ring is in the 3 position and includes all possible configurations of the asymmetric carbon.

7. A compound according to claim 6, wherein the carbon atom at the 3-position of the azoniabicyclic group has R configuration.

8. A compound according to claim 6, wherein the carbon atom at the 3-position of the azoniabicyclic group has S configuration.

9. A compound according to any preceding claim wherein D represents a group of formula (i) and R⁵ represents an unsubstituted phenyl, 2-thienyl, 3-thienyl, 2-furyl or 3-furyl group.

10. A compound according to any preceding claim wherein D represents a group of formula (i) and R⁶ represents a 2-thienyl, 3-thienyl, 2-furyl, 3-furyl or cyclopentyl group.

11. A compound according to any preceding claim wherein D represents a group of formula (i) and wherein the group -O-CO-C(R⁵)(R⁶)(R⁷) represents a group selected from 2,2-dithien-2-ylacetoxy, 2,2-dithien-2-ylpropionyloxy, 2-hydroxy-2,2-dithien-2-ylacetoxy, 2-hydroxy-2-phenyl-2-thien-2-ylacetoxy, 2-fur-2-yl-2-hydroxy-2-phenylacetoxy, 2-fur-2-yl-2-hydroxy-2-thien-2-ylacetoxy, (2*)-2-hydroxy-2,3-diphenylpropionyloxy, 2-hydroxy-2-thien-2-ylpent-4-enoyloxy, (2S)-2-cyclopentyl-2-hydroxy-2-thien-2-ylacetoxy and (2R)-2-cyclopentyl-2-hydroxy-2-thien-2-ylacetoxy.

12. A compound according to claim 11 wherein the group -O-CO-C(R⁵)(R⁶)(R⁷) represents a group selected from 2-cyclopentyl-2-hydroxy-2-thien-2-ylacetoxy; 2,2-dithien-2-ylacetoxy, 2-hydroxy-2,2-dithien-2-ylacetoxy, 2,2-dithien-2-ylpropionyloxy; 2-hydroxy-2-phenyl-2-thien-2-ylacetoxy, '2-fur-2-yl-2-hydroxy-2-thien-2-ylacetoxy and 2-fur-2-yl-2-hydroxy-2-phenylacetoxy.

13. A compound according to any preceding claim wherein D represents a group of formula (ii) and wherein the group D-COO- represents a group selected from 9-methyl-9H-fluorene-9-carbonyloxy, 9-hydroxy-9H-fluorene-9-carbonyloxy, 9H-xanthene-9-carbonyloxy, 9-methyl-9H-xanthene-9-carbonyloxy, 9-hydroxy-9H-xanthene-9-carbonyloxy 9,10-dihydroanthracene-9-carbonyloxy and 10,11-dihydro-5H-dibenzo[a,d]cycloheptene-5-carbonyloxy.

14. A compound according to claim 13 wherein the group D-COO- represents a group selected from 9-methyl-9H-fluorene-9-carbonyloxy, 9-hydroxy-9H-fluorene-9-carbonyloxy, 9H-xanthene-9-carbonyloxy, 9-methyl-9H-xanthene-9-carbonyloxy and 9-hydroxy-9H-xanthene-9-carbonyloxy.

15. A compound according to any one of the preceding claims, wherein the carbon substituted by R⁵, R⁶ and R⁷ has R configuration.

16. A compound according to any one of claims 1 to 14 wherein the carbon substituted by R⁵, R⁶ and R⁷ has S configuration.

17. A compound according to claim 1 which is:
(3R)-1-Allyl-3-(2,2-dithien-2-ylacetoxy)-1-azoniabicyclo[2.2.2]octane bromide
(3R)-3-(2,2-Dithien-2-ylacetoxy)-1-(4-methylpent-3-enyl)-1-azoniabicyclo[2.2.2]octane bromide
(3R)-1-Allyl-3-(2,2-dithien-2-ylpropionyloxy)-1-azoniabicyclo[2.2.2]octane bromide
(3R)-1-(4-Methylpent-3-enyl)-3-(2,2-dithien-2-ylpropionyloxy)-1-azoniabicyclo[2.2.2]octane bromide
(3R)-3-(2-Hydroxy-2, 2-dithien-2-ylacetoxy)-1-isopropyl-1-azoniabicyclo[2.2.2]octane trifluoroacetate
(3R)-1-Cyclopropylmethyl-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-isobutyl-1-azoniabicyclo[2.2.2]octane trifluoroacetate
(3R)-1-Heptyl-3-(2-hydroxy-2,2-dithien-2-ylacetoxy)-1-azoniabicyclo[2.2.2]octane bromide
(3R)-1-Cyclohexylmethyl-3-(2-hydroxy-2,2-dithien-2-ylacetoxy)-1-azonabicyclo[2.2.2]octane trifluoroacetate
(3R)-1-(3-Cyclohexylpropyl)-3-(2-hydroxy-2,2-dithien-2-ylacetoxy)-1-azoniabicyclo[2.2.2]octane trifluoroacetate
(3R)-1-Allyl-3-(2-hydroxy-2, 2-dithien-2-ylacetoxy)-1-azoniabicyclo[2.2.2]octane bromide
(3R)-3-(2-Hydroxy-2,2-dithien-2-ylacetoxy)-1-(4-methylpent-3-enyl)-1-azoniabicyclo[2.2.2]octane bromide
(3R)-1-(3,7-Dimethylocta-(E)-2,6-dienyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-(2-hydroxyethyl)-1-azoniabicyclo[2.2.2]octane trifluoroacetate
(3R)-3-(2-Hydroxy-2,2-dithien-2-ylacetoxy)-1-(3-hydroxypropyl)-1-azoniabicyclo[2.2.2]octane trifluoroacetate
(3R)-1-(4-Hydroxybutyl)-3-(2-hydroxy-2,2-dithien-2-ylacetoxy)-1-azoniabicyclo[2.2.2]octane trifluoroacetate
(3R)-1-(2-Ethoxyethyl)-3-(2-hydroxy-2,2-dithien-2-ylacetoxy)-1-azoniabicyclo[2.2.2]octane bromide
(3R)-3-(2-Hydroxy-2,2-dithien-2-ylacetoxy)-1-[2-(2-hydroxyethoxy)ethyl]-1-azoniabicyclo[2.2.2]octane chloride
(3R)-3-(2-Hydroxy-2,2-dithien-2-ylacetoxy)-1-[2-(2-methoxyethoxy)ethyl]-1-azoniabicyclo[2.2.2]octane bromide
(3R)-3-(2-Hydroxy-2,2-dithien-2-yl-acetoxy)-1-oxiranylmethyl-1-azoniabicyclo[2.2.2]octane bromide
(3R)-1-(2-[1,3]Dioxolan-2-ylethyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-{2-[2-(2-hydroxyethoxy)-ethoxy]ethyl}-1-azoniabicyclo[2.2.2]octane trifluoroacetate
(3R)-1-(3-[1,3]Dioxolan-2-ylpropyl)-3-(2-hydroxy-2,2-dithien-2-ylacetoxy)-1-azoniabicyclo[2.2.2]octane trifluoroacetate
(3R)-1-(3-Ethoxycarbonylpropyl)-3-(2-hydroxy-2,2-dithien-2-ylacetoxy)-1-azoniabicyclo[2.2.2]octane trifluoroacetate
(3R)-1-(4-Ethoxycarbonylbutyl)-3-(2-hydroxy-2,2-dithien-2-ylacetoxy)-1-azoniabicyclo[2.2.2]octane trifluoroacetate
(3R)-1-(4-Acetoxybutyl)-3-(2-hydroxy-2,2-dithien-2-ylacetoxy)-1-azoniabicyclo[2.2.2]octane trifluoroacetate
(3R)-1-(3-Cyanopropyl)-3-(2-hydroxy-2,2-dithien-2-ylacetoxy)-1-azoniabicyclo[2.2.2]octane trifluoroacetate
(3R)-1-(4-Cyanobutyl)-3-(2-hydroxy-2,2-dithien-2-ylacetoxy)-1-azoniabicyclo[2.2.2]octane trifluoroacetate
(3R)-1-(6-Cyanohexyl)-3-(2-hydroxy-2,2-dithien-2-ylacetoxy)-1-azoniabicyclo[2.2.2]octane trifluoroacetate
(3R)-3-(2-Hydroxy-2,2-dithien-2-ylacetoxy)-1-(4,4,4-trifluorobutyl)-1-azoniabicyclo[2.2.2]octane trifluoroacetate
(3R)-1-Allyl-3-(2-hydroxy-2-phenyl-2-thien-2-ylacetoxy)-1-azoniabicyclo[2.2.2]octane bromide
(3R)-1-Allyl-3-(2-fur-2-yl-2-hydroxy-2-phenylacetoxy)-1-azoniabicyclo[2.2.2]octane bromide
(3R)-1-Allyl-3-(2-fur-2-yl-2-hydroxy-2-thien-2-ylacetoxy)-1-azoniabicyclo[2.2.2]octane bromide
(3R)-1-Allyl-3-(9-methyl-9H-fluorene-9-carbonyloxy)-1-azoniabicyclo[2.2.2]octane bromide
(3R)-1-Allyl-3-(9-hydroxy-9H-fluorene-9-carbonyloxy)-1-azoniabicyclo[2.2.2]octane bromide
(3R)-3-(9-Hydroxy-9H-fluorene-9-carbonyloxy)-1-(4-methylpent-3-enyl)-1-azoniabicyclo[2.2.2]octane bromide
(3R)-1-Heptyl-3-(9-hydroxy-9H-fluorene-9-carbonyloxy)-1-azoniabicyclo[2.2.2]octane bromide
(3R)-3-(9-Hydroxy-9H-fluorene-9-carbonyloxy)-1-oxiranylmethyl-1-azoniabicyclo[2.2.2]octane bromide
(3R)-3-(9-Hydroxy-9H-fluorene-9-carbonyloxy)-1-[2-(2-methoxyethoxy)ethyl]-1-azoniabicyclo[2.2.2]octane bromide
(3R)-1-(2-[1,3]Dioxolan-2-ylethyl)-3-(9-hydroxy-9H-fluorene-9-carbonyloxy)-1-azoniabicyclo[2.2.2]octane bromide
(3R)-1-Allyl-3-(9H-xanthene-9-carbonyloxy)-1-azoniabicyclo[2.2.2]octane bromide
(3R)-1-(4-Methylpent-3-enyl)-3-(9H-xanthene-9-carbonyloxy)-1-azoniabicyclo[2.2.2]octane bromide
(3R)-1-Allyl-3-(9-methyl-9H-xanthene-9-carbonyloxy)-1-azoniabicyclo[2.2.2]octane bromide
(3R)-1-Allyl-3-(9-hydroxy-9H-xanthene-9-carbonyloxy)-1-azoniabicyclo[2.2.2]octane bromide
(3R)-1-(3-Hydroxypropyl)-3-(9-hydroxy-9H-xanthene-9-carbonyloxy)-1-azoniabicyclo[2.2.2]octane bromide
(3R)-1-Allyl-3-(10,11-dihydro-5H-dibenzo[a,d]cycloheptene-5-carbonyloxy)-1-azoniabicyclo[2.2.2]octane bromide
(3R)-1-(4-Methylpent-3-enyl)-3-(10,11-dihydro-5H-dibenzo[a,d]cycloheptene-5-carbonyloxy)-1-azoniabicyclo[2.2.2]octane bromide
(3R)-1-Allyl-3-[(2*)-2-hydroxy-2,3-diphenylpropionyloxy]-1-azoniabicyclo[2.2.2]octane bromide
(3R)-3-[(2*)-2-Hydroxy-2,3-diphenylpropionyloxy)]-1-(4-methylpent-3-enyl)-1-azoniabicyclo[2.2.2]octane bromide
(3R)-1-Allyl-3-(2-hydroxy-2-thien-2-ylpent-4-enoyloxy)-1-azoniabicyclo[2.2.2]octane bromide
(3R)-1-(4-Methylpent-3-enyl)-3-(2-Hydroxy-2-thien-2-ylpent-4-enoyloxy)-1-azoniabicyclo[2.2.2]octane bromide
(3R)-1-Allyl-3-[(2S)-2-cyclopentyl-2-hydroxy-2-thien-2-ylacetoxy]-1-azoniabicyclo[2.2.2]octane bromide
(3R)-1-(4-Methylpent-3-enyl)-3-[(2S)-2-cyclopentyl-2-hydroxy-2-thien-2-ylacetoxy)-1-azoniabicyclo[2.2.2]octane bromide
(3R)-1-Allyl-3-[(2R)-2-cyclopentyl-2-hydroxy-2-thien-2-ylacetoxy)-1-azoniabicyclo[2.2.2]octane bromide
(3R)-3-[(2S)-2-Cyclopentyl-2-hydroxy-2-thien-2-ylacetoxy]-1-(2-hydroxyethyl)-1-azoniabicyclo[2.2.2]octane bromide
(3R)-3-[(2S)-2-Cyclopentyl-2-hydroxy-2-thien-2-ylacetoxy]-1-(3-hydroxypropyl)-1-azoniabicyclo[2.2.2]octane bromide
(3R)-3-[(2S)-2-Cyclopentyl-2-hydroxy-2-thien-2-ylacetoxy]-1-(4-hydroxybutyl)-1-azoniabicyclo[2.2.2]octane bromide
(3R)-3-[(2S)-2-Cyclopentyl-2-hydroxy-2-thien-2-ylacetoxy]-1-[2-(2-hydroxyethoxy)ethyl]-1-azoniabicyclo[2.2.2]octane bromide
(3R)-3-[(2S)-2-Cyclopentyl-2-hydroxy-2-thien-2-ylacetoxy]-1-(6-hydroxyhexyl)-1-azoniabicyclo[2.2.2]octane bromide
(3R)-3-[(2S)-2-Cyclopentyl-2-hydroxy-2-thien-2-ylacetoxy]-1-(5-hydroxypentyl)-1-azoniabicyclo[2.2.2]octane bromide
(3R)-3-[(2S)-2-Cyclopentyl-2-hydroxy-2-thien-2-ylacetoxy]-1-[3-(4-hydroxypiperidin-1-yl)propyl]-1-azoniabicyclo[2.2.2]octane bromide
(3R)-3-[(2S)-2-Cyclopentyl-2-hydroxy-2-thien-2-ylacetoxy]-1-[4-(4-hydroxypiperidin-1-yl)butyl]-1-azoniabicyclo[2.2.2]octane bromide
(3R)-3-(9,10-Dihydroanthracene-9-carbonyloxy)-1-(2-hydroxyethyl)-1-azoniabicyclo[2.2.2]octane bromide
(3R)-1-(2-Hydroxyethyl)-3-(9H-xanthene-9-carbonyloxy)-1-azoniabicyclo[2.2.2]octane bromide
(3R)-1-(2-Hydroxyethyl)-3-(9-hydroxy-9H-xanthene-9-carbonyloxy)-1-azoniabicyclo[2.2.2]octane bromide
(3R)-1-(2-Hydroxyethyl)-3-(9-hydroxy-9H-fluorene-9-carbonyloxy)-1-azoniabicyclo[2.2.2]octane bromide
(3R)-1-(2-Hydroxyethyl)-3-(9-methyl-9H-fluorene-9-carbonyloxy)-1-azoniabicyclo[2.2.2]octane bromide

18. A process for producing compounds of formula (I). 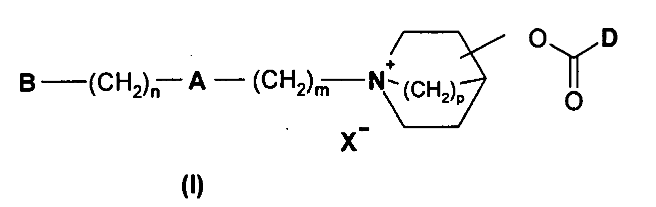 wherein B, n, A, m, p, and D are as defined in any of claims 1 to 17, which process comprises quaternising the nitrogen atom of the azabicyclic ring of a compound of formula (III): 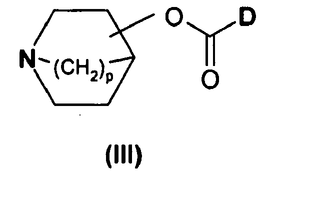 wherein p and D are as defined above, with an alkylating agent of formula (II): 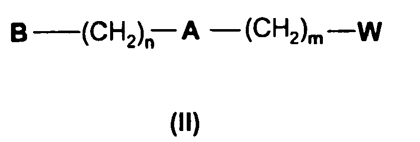 wherein B, n, A and m are as defined above, and W represents a leaving group.

19. A pharmaceutical composition comprising a compound according to any one of claims 1 to 17 in admixture with a pharmaceutically acceptable carrier or diluent.

20. A compound according to any one of claims 1 to 17, or a pharmaceutical composition according to claim 19, for therapeutic use in a method of treatment of the human or animal body.

21. Use of a compound according to any one of claims 1 to 17, or a pharmaceutical composition according to claim 19, in the manufacture of a medicament for the treatment of a respiratory, urological or gastrointestinal disease or disorder.

22. Use according to claim 21, wherein the disease or disorder is selected from the group consisting of chronic obstructive pulmonary disease (COPD), bronchitis, bronchial hyperreactivity, asthma, cough, rhinitis, urinary incontinence, pollakiuria, neurogenic or unstable bladder, cytospasm, chronic cystitis, irritable bowel syndrome, spastic colitis, diverticulitis and peptic ulceration.

23. A combination product comprising
(i) a compound according to any one of claims 1 to 17; and
(ii) another compound effective in the treatment of a respiratory, urological or gastrointestinal disease or disorder
for simultaneous, separate or sequential use.

24. A combination product according to claim 23 comprising
(i) a compound according to any one of claims 1 to 17; and
(ii) a β₂ agonist, steroid, antiallergic drug, phosphodiesterase IV inhibitor and/or leukoteriene D4 (LTD4) antagonist
for simultaneous, separate or sequential use in the treatment of a respiratory disease.

## Patentansprüche

1. Verbindung von Formel (I): 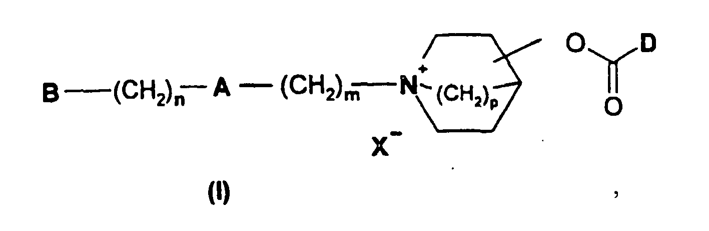 worin B für ein Wasserstoffatom oder eine Gruppe steht, die ausgewählt ist aus -R¹, -OR¹, Hydroxy, -O(CO)R¹, Cyano und einem gegebenenfalls substituierten nicht-aromatischen Heterocyclyl, das ein oder mehrere Heteroatome enthält, wobei
R¹ ausgewählt ist aus der Gruppe, die aus Wasserstoffatomen, gegebenenfalls substituiertem C₁₋₈-Alkyl, gegebenenfalls substituiertem C₂₋₈-Alkenyl und gegebenenfalls substituiertem C₃-C₈-Cycloalkyl besteht;
n eine ganze Zahl von 0 bis 4 ist;
A für eine Gruppe steht, die ausgewählt ist aus -CH₂-, -CH=CR³-, -CR³=CH-, -CR³R⁴-, -O-, -CO-, -O-(CH₂)₂-O-, wobei R³ und R⁴ jeweils unabhängig für ein Wasserstoffatom oder eine C₁₋₈-Alkylgruppe stehen;
m eine ganze Zahl von 0 bis 8 ist;
p eine ganze Zahl von 1 bis 2 ist
und die Substitution im bicyclischen Azoniaring in der 2-, 3- oder 4-Position vorliegen kann, einschließlich aller möglichen Konfigurationen der asymmetrischen Zentren;
D eine Gruppe ist, die ausgewählt ist aus: 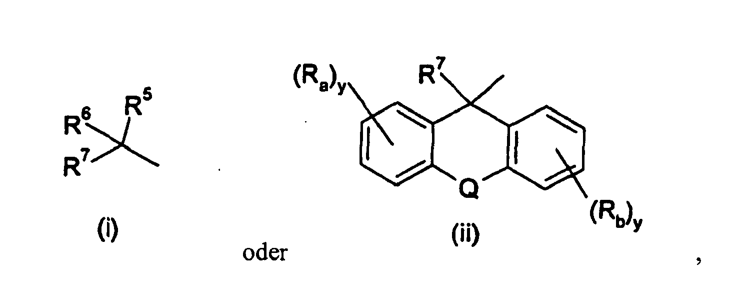 wobei R⁵ für eine Gruppe steht, die ausgewählt ist aus Phenyl, 2-Thienyl, 3-Thienyl, 2-Furanyl, 3-Furanyl, wobei diese Gruppe gegebenenfalls mit einem oder mehreren Substituenten Rₐ substituiert sein kann;
R⁶ für eine Gruppe steht, die ausgewählt ist aus 2-Thienyl, 3-Thienyl, 2-Furanyl, 3-Furanyl, C₃-C₈-Cycloalkyl, C₃-C₈-Cycloalkenyl, C₁₋₈-Alkyl, C₂₋₈-Alkenyl, C₂₋₈-Alkinyl, Benzyl und Phenylethyl, wobei diese Gruppe gegebenenfalls mit einem oder mehreren Substituenten R_{b} substituiert sein kann;
R⁷ für ein Wasserstoffatom oder eine Gruppe steht, die ausgewählt ist aus Hydroxyl, Hydroxymethyl und Methyl;
Q für eine Einfachbindung oder eine Gruppe steht, die ausgewählt ist aus -CH₂-, -CH₂CH₂-, -O-, -O-CH₂-, -S-, -S-CH₂- und -CH=CH-;
Rₐ und R_{b} unabhängig für eine Gruppe stehen, die ausgewählt ist aus Halogenatomen, gegebenenfalls substituiertem C₁₋₈-Alkyl, gegebenenfalls substituiertem C₁₋₈-Alkoxy, Hydroxy, Trifluormethyl, Nitro, Cyano, -COOR⁸, -NR⁸R⁹, wobei R⁸ und R⁹ unabhängig für ein Wasserstoffatom oder eine C₁₋₈-Alkylgruppe stehen;
y eine ganze Zahl von 0 bis 3 ist;
X⁻ für ein pharmazeutisch verträgliches Anion einer ein- oder mehrwertigen Säure steht;
vorausgesetzt, dass die Gruppe B-(CH₂)ₙ-A-(CH₂)ₘ- nicht eine lineare C₁₋₄-Alkylgruppe ist, und weiter vorausgesetzt, dass die Verbindung nicht eine von:
1-Allyloxycarbonylmethyl-3-(2-hydroxy-2,2-di-thiophen-2-yl-acetoxy)-1-azonia-bicyclo-[2.2.2]octan; und
1-Carboxymethyl-3-(2-hydroxy-2,2-di-thiophen-2-yl-acetoxy)-1-azonia-bicyclo-[2.2.2]octan
ist.

2. Verbindung nach Anspruch 1, wobei B ausgewählt ist aus der Gruppe, die aus Wasserstoffatomen, Hydroxygruppen, gegebenenfalls substituierten C₁₋₈-Alkyl-, gegebenenfalls substituierten C₂₋₈-Alkenyl-, gegebenenfalls substituierten C₃-C₈-Cycloalkylgruppen und nicht-aromatischen Heterocyclylgruppen, die wenigstens mit einer Hydroxygruppe substituiert sind, besteht.

3. Verbindung nach Anspruch 1, wobei die Azoniabicyclogruppe am Stickstoffatom mit einer Gruppe substituiert ist, die ausgewählt ist aus Allyl, 4-Methylpent-3-enyl, Isopropyl, Cyclopropylmethyl, Isobutyl, Heptyl, Cyclohexylmethyl, 3-Cyclohexylpropyl, 3,7-Dimethylocta-(E)-2,6-dienyl, 2-Hydroxyethyl, 3-Hydroxypropyl, 4-Hydroxybutyl, 5-Hydroxypentyl, 6-Hydroxyhexyl, 2-Ethoxyethyl, 2-(2-Hydroyethoxy)ethyl, 2-(2-Methoxyethoxy)ethyl, Oxiranylmethyl, 2-[1,3]Dioxolan-2-ylethyl, 2-[2-(2-Hydroxyethoxy)-ethoxy]ethyl, 3-[1,3]Dioxolan-2-ylpropyl, 2-Ethoxycarbonylethyl, 3-Ethoxycarbonylpropyl, 4-Ethoxycarbonylbutyl, 4-Acetoxybutyl, 2-Cyanoethyl, 3-Cyanopropyl, 4-Cyanobutyl, 6-Cyanohexyl, 4,4,4-Trifluorbutyl, 3-(4-Hydroxypiperidin-1-yl)propyl und 4-(4-Hydroxypiperidin-1-yl)butyl.

4. Verbindung nach Anspruch 3, wobei die Azoniabicyclogruppe am Stickstoffatom mit einer Gruppe substituiert ist, die ausgewählt ist aus Allyl, 4-Methylpent-3-enyl, Isopropyl, Cyclopropylmethyl, Isobutyl, Heptyl, 2-Hydroxyethyl, 3-Hydroxypropyl, 4-Hydroxybutyl, 5-Hydroxypentyl, 6-Hydroxyhexyl, 2-(2-Methoxyethoxy)ethyl, 2-(2-Hydroxyethoxy)ethyl, 4-Ethoxycarbonylbutyl, 4-Acetoxybutyl, 3-Cyanopropyl und 4-Cyanobutyl.

5. Verbindung nach einem vorangehenden Anspruch, wobei p 2 ist.

6. Verbindung nach einem vorangehenden Anspruch, wobei die Substitution in dem bicyclischen Azoniaring in der 3-Position vorliegt und alle möglichen Konfigurationen des asymmetrischen Kohlenstoffes einschließt.

7. Verbindung nach Anspruch 6, wobei das Kohlenstoffatom an der 3-Position der Azoniabicyclogruppe R-Konfiguration aufweist.

8. Verbindung nach Anspruch 6, wobei das Kohlenstoffatom an der 3-Position der Azoniabicylogruppe S-Konfiguration aufweist.

9. Verbindung nach einem vorangehenden Anspruch, wobei D für eine Gruppe von Formel (i) steht und R⁵ für eine unsubstituierte Phenyl-, 2-Thienyl-, 3-Thienyl-, 2-Furyl- oder 3-Furylgruppe steht.

10. Verbindung nach einem vorangehenden Anspruch, wobei D für eine Gruppe von Formel (i) steht und R⁶ für eine 2-Thienyl-, 3-Thienyl-, 2-Furyl-, 3-Furyl- oder Cyclopentylgruppe steht.

11. Verbindung nach einem vorangehenden Anspruch, wobei D für eine Gruppe von Formel (i) steht und wobei die Gruppe -O-CO-C(R⁵)(R⁶)(R⁷) für eine Gruppe steht, die ausgewählt ist aus 2,2-Dithien-2-ylacetoxy, 2,2-Dithien-2-ylpropionyloxy, 2-Hydroxy-2,2-dithien-2-ylacetoxy, 2-Hydroxy-2-phenyl-2-thien-2-ylacetoxy, 2-Fur-2-yl-2-hydroxy-2-phenylacetoxy, 2-Fur-2-yl-2-hydroxy-2-thien-2-ylacetoxy, (2*)-2-Hydroxy-2,3-diphenylpropionyloxy, 2-Hydroxy-2-thien-2-ylpent-4-enoyloxy, (2S)-2-Cyclopentyl-2-hydroxy-2-thien-2-ylacetoxy und (2R)-2-Cyclopentyl-2-hydroxy-2-thien-2-ylacetoxy.

12. Verbindung nach Anspruch 11, wobei die Gruppe -O-CO-C(R⁵)(R⁶)(R⁷) für eine Gruppe steht, die ausgewählt ist aus 2-Cyclopentyl-2-hydroxy-2-thien-2-ylacetoxy; 2,2-Dithien-2-ylacetoxy, 2-Hydroxy-2,2-dithien-2-ylacetoxy, 2,2-Dithien-2-ylpropionyloxy; 2-Hydroxy-2-phenyl-2-thien-2-ylacetoxy, 2-Fur-2-yl-2-hydroxy-2-thien-2-ylacetoxy und 2-Fur-2-yl-2-hydroxy-2-phenylacetoxy.

13. Verbindung nach einem vorangehenden Anspruch, wobei D für eine Gruppe von Formel (ii) steht und wobei die Gruppe D-COO- für eine Gruppe steht, die ausgewählt ist aus 9-Methyl-9H-fluoren-9-carbonyloxy, 9-Hydroxy-9H-fluoren-9-carbonyloxy, 9H-Xanthen-9-carbonyloxy, 9-Methyl-9H-xanthen-9-carbonyloxy, 9-Hydroxy-9H-xanthen-9-carbonyloxy, 9,10-Dihydroanthracen-9-carbonyloxy und 10,11-Dihydro-5H-dibenzo[a,d]cyclohepten-5-carbonyloxy.

14. Verbindung nach Anspruch 13, wobei die Gruppe D-COO- für eine Gruppe steht, die ausgewählt ist aus 9-Methyl-9H-fluoren-9-carbonyloxy, 9-Hydroxy-9H-fluoren-9-carbonyloxy, 9H-Xanthen-9-carbonyloxy, 9-Methyl-9H-xanthen-9-carbonyloxy und 9-Hydroxy-9H-xanthen-9-carbonyloxy.

15. Verbindung nach einem der vorangehenden Ansprüche, wobei der mit R⁵, R⁶ und R⁷ substituierte Kohlenstoff R-Konfiguration aufweist.

16. Verbindung nach einem der Ansprüche 1 bis 14, wobei der mit R⁵, R⁶ und R⁷ substituierte Kohlenstoff S-Konfiguration aufweist.

17. Verbindung nach Anspruch 1, die :
(3R)-1-Allyl-3-(2,2-dithien-2-ylacetoxy)-1-azoniabicyclo[2.2.2]octan-Bromid
(3R)-3-(2,2-Dithien-2-ylacetoxy)-1-(4-methylpent-3-enyl)-1-azoniabicyclo[2.2.2]-octan-Bromid
(3R)-1-Allyl-3-(2,2-dithien-2-ylpropionyloxy)-1-azoniabicyclo[2.2.2]octan-Bromid
(3R)-1-(4-Methylpent-3-enyl)-3-(2,2-dithien-2-ylpropionyloxy)-1-azoniabicyclo-[2.2.2]octan-Bromid
(3R)-3-(2-Hydroxy-2,2-dithien-2-ylacetoxy)-1-isopropyl-1-azoniabicyclo[2.2.2]-octan-Trifluoracetat
(3R)-1-Cyclopropylmethyl-3-(2-hydroxy-2,2-dithien-2-ylacetoxy)-1-azoniabicyclo-[2.2.2]octan-Trifluoracetat
(3R)-3-(2-Hydroxy-2,2-dithien-2-ylacetoxy)-1-isobutyl-1-azoniabicyclo[2.2.2]-octan-Trifluoracetat
(3R)-1-Heptyl-3-(2-hydroxy-2,2-dithien-2-ylacetoxy)-1-azoniabicyclo[2.2.2]-octan-Bromid
(3R)-1-Cyclohexylmethyl-3-(2-hydroxy-2,2-dithien-2-ylacetoxy)-1-azoniabicyclo-[2.2.2]octan-Trifluoracetat
(3R)-1-(3-Cyclohexylpropyl)-3-(2-hydroxy-2,2-dithien-2-ylacetoxy)-1-azoniabicyclo-[2.2.2]octan-Trifluoracetat
(3R)-1-Allyl-3-(2-hydroxy-2,2-dithien-2-ylacetoxy)-1-azoniabicyclo[2.2.2]-octan-Bromid
(3R)-3-(2-Hydroxy-2,2-dithien-2-ylacetoxy)-1-(4-methylpent-3-enyl)-1-azoniabicyclo[2.2.2]octan-Bromid
(3R)-1-(3,7-Dimethylocta-(E)-2,6-dienyl)-3-(2-hydroxy-2,2-dithien-2-ylacetoxy)-1-azoniabicyclo[2.2.2]octan-Trifluoracetat
(3R)-3-(2-Hydroxy-2,2-dithien-2-ylacetoxy)-1-(2-hydroxyethyl)-1-azoniabicyclo-[2.2.2]octan-Trifluoracetat
(3R)-3-(2-Hydroxy-2,2-dithien-2-ylacetoxy)-1-(3-hydroxypropyl)-1-azoniabicyclo-[2.2.2]octan-Trifluoracetat
(3R)-1-(4-Hydroxybutyl)-3-(2-hydroxy-2.2-dithien-2-ylacetoxy)-1-azoniabicyclo-[2.2.2]octan-Trifluoracetat
(3R)-1-(2-Ethoxyethyl)-3-(2-hydroxy-2,2-dithien-2-ylacetoxy)-1-azoniabicyclo-[2.2.2]octan-Bromid
(3R)-3-(2-Hydroxy-2,2-dithien-2-ylacetoxy)-1-[2-(2-hydroxyethoxy)ethyl]-1-azoniabicyclo[2.2.2]octan-Chlorid
(3R)-3-(2-Hydroxy-2,2-dithien-2-ylacetoxy)-1-[2-(2-methoxyethoxy)ethyl]-1-azoniabicyclo[2.2.2]octan-Bromid
(3R)-3-(2-Hydroxy-2,2-dithien-2-yl-acetoxy)-1-oxiranylmethyl-1-azoniabicyclo-[2.2.2]octan-Bromid
(3R)-1-(2-[1,3]Dioxolan-2-ylethyl)-3-(2-hydroxy-2,2-dithien-2-ylacetoxy)-1-azoniabicyclo[2.2.2]octan-Trifluoracetat
(3R)-3-(2-Hydroxy-2,2-dithien-2-ylacetoxy)-1-{2-[2-(2-hydroxyethoxy)-ethoxy]-ethyl}-1-azoniabicyclo[2.2.2]octan-Trifluoracetat
(3R)-1-(3-[1,3]Dioxolan-2-ylpropyl)-3-(2-hydroxy-2,2-dithien-2-ylacetoxy)-1-azoniabicyclo[2.2.2]octan-Trifluoracetat
(3R)-1-(3-Ethoxycarbonylpropyl)-3-(2-hydroxy-2,2-dithien-2-ylacetoxy)-1-azoniabicyclo[2.2.2]octan-Trifluoracetat
(3R)-1-(4-Ethoxycarbonylbutyl)-3-(2-hydroxy-2,2-dithien-2-ylacetoxy)-1-azoniabicyclo[2.2.2]octan-Trifluoracetat
(3R)-1-(4-Acetoxybutyl)-3-(2-hydroxy-2,2-dithien-2-ylacetoxy)-1-azoniabicyclo [2.2.2]octan-Trifluoracetat
(3R)-1-(3-Cyanopropyl)-3-(2-hydroxy-2,2-dithien-2-ylacetoxy)-1-azoniabicyclo[2.2.2]octan-Trifluoracetat
(3R)-1-(4-Cyanobutyl)-3-(2-hydroxy-2,2-dithien-2-ylacetoxy)-1-azoniabicyclo[2.2.2]octan-Trifluoracetat
(3R)-1-(6-Cyanohexyl)-3-(2-hydroxy-2,2-dithien-2-ylacetoxy)-1-azoniabicyclo[2.2.2]octan-Trifluoracetat
(3R)-3-(2-Hydroxy-2,2-dithien-2-ylacetoxy)-1-(4,4,4-trifluorbutyl)-1-azoniabicyclo[2.2.2]octan-Trifluoracetat
(3R)-1-Allyl-3-(2-hydroxy-2-phenyl-2-thien-2-ylacetoxy)-1-azoniabicyclo[2.2.2]octan-Bromid
(3R)-1-Allyl-3-(2-fur-2-yl-2-hydroxy-2-phenylacetoxy)-1-azoniabicyclo[2.2.2]octan-Bromid
(3R)-1-Allyl-3-(2-fur-2-yl-2-hydroxy-2-thien-2-ylacetoxy)-1-azoniabicyclo[2.2.2]octan-Bromid
(3R)-1-Allyl-3-(9-methyl-9H-fluoren-9-carbonyloxy)-1-azoniabicyclo[2.2.2]octan-Bromid
(3R)-1-Allyl-3-(9-hydroxy-9H-fluoren-9-carbonyloxy)-1-azoniabicyclo[2.2.2]octan-Bromid
(3R)-3-(9-Hydroxy-9H-fluoren-9-carbonyloxy)-1-(4-methylpent-3-enyl)-1-azoniabicyclo[2.2.2]octan-Bromid
(3R)-1-Heptyl-3-(9-hydroxy-9H-fluoren-9-carbonyloxy)-1-azoniabicyclo[2.2.2]octan-Bromid
(3R)-3-(9-Hydroxy-9H-fluoren-9-carbonyloxy)-1-oxiranylmethyl-1-azoniabicyclo[2.2.2]octan-Bromid
(3R)-3-(9-Hydroxy-9H-fluoren-9-carbonyloxy)-1-[2-(2-methoxyethoxy)ethyl]-1-azoniabicyclo[2.2.2]octan-Bromid
(3R)-1-(2-[1,3]Dioxolan-2-ylethyl)-3-(9-hydroxy-9H-fluoren-9-carbonyloxy)-1-azoniabicyclo[2.2.2]octan-Bromid
(3R)-1-Allyl-3-(9H-xanthen-9-carbonyloxy)-1-azoniabicyclo[2.2.2]octan-Bromid
(3R)-1-(4-Methylpent-3-enyl)-3-(9H-xanthen-9-carbonyloxy)-1-azoniabicyclo[2.2.2]octan-Bromid
(3R)-1-Allyl-3-(9-methyl-9H-xanthen-9-carbonyloxy)-1-azoniabicyclo[2.2.2]octan-Bromid
(3R)-1-Allyl-3-(9-hydroxy-9H-xanthen-9-carbonyloxy)-1-azoniabicyclo[2.2.2]octan-Bromid
(3 R)-1-(3 -Hydroxypropyl)-3-(9-hydroxy-9H-xanthen-9-carbonyloxy)-1-azoniabicyclo[2.2.2]octan-Bromid
(3R)-1-Allyl-3-(10,11-dihydro-5H-dibenzo[a,d]cyclohepten-5-carbonyloxy)-1-azoniabicyclo[2.2.2]octan-Bromid
(3R)-1-(4-Methylpent-3-enyl)-3-(10,11-dihydro-5H-dibenzo[a,d]cyclohepten-5-carbonyloxy)-1-azoniabicyclo[2.2.2]octan-Bromid
(3R)-1-Allyl-3-[(2*)-2-hydroxy-2,3-diphenylpropionyloxy]-1-azoniabicyclo[2.2.2]octan-Bromid
(3R)-3-[(2*)-2-Hydroxy-2,3-diphenylpropionyloxy]-1-(4-methylpent-3-enyl)-1-azoniabicyclo[2.2.2]octan-Bromid
(3R)-1-Allyl-3-(2-hydroxy-2-thien-2-ylpent-4-enoyloxy)-1-azoniabicyclo[2.2.2]octan-Bromid
(3 R)-1-(4-Methylpent-3-enyl)-3 -(2-hydroxy-2-thien-2-ylpent-4-enoyloxy)-1-azoniabicyclo[2.2.2]octan-Bromid
(3R)-1-Allyl-3-[(2S)-2-cyclopentyl-2-hydroxy-2-thien-2-ylacetoxy]-1-azoniabicyclo[2.2.2]octan-Bromid
(3R)-1-(4-Methylpent-3-enyl)-3-[(2S)-2-cyclopentyl-2-hydroxy-2-thien-2-ylacetoxy]-1-azoniabicyclo[2.2.2]octan-Bromid
(3R)-1-Allyl-3-[(2R)-2-cyclopentyl-2-hydroxy-2-thien-2-ylacetoxy]-1-azoniabicyclo[2.2.2]octan-Bromid
(3R)-3-[(2S)-2-Cyclopentyl-2-hydroxy-2-thien-2-ylacetoxy]-1-(2-hydroxyethyl)-1-azoniabicyclo[2.2.2]octan-Bromid
(3R)-3-[(2S)-2-Cyclopentyl-2-hydroxy-2-thien-2-ylacetoxy]-1-(3-hydroxypropyl)-1-azoniabicyclo[2.2.2]octan-Bromid
(3R)-3-[(2S)-2-Cyclopentyl-2-hydroxy-2-thien-2-ylacetoxy]-1-(4-hydroxybutyl)-1-azoniabicyclo[2.2.2]octan-Bromid
(3R)-3-[(2S)-2-Cyclopentyl-2-hydroxy-2-thien-2-ylacetoxy]-1-[2-(2-hydroxyethoxy)ethyl]-1-azoniabicyclo[2.2.2]octan-Bromid
(3R)-3-[(2S)-2-Cyclopentyl-2-hydroxy-2-thien-2-ylacetoxy]-1-(6-hydroxyhexyl)-1-azoniabicyclo[2.2.2]octan-Bromid
(3R)-3-[(2S)-2-Cyclopentyl-2-hydroxy-2-thien-2-ylacetoxy]-1-(5-hydroxypentyl)-1-azoniabicyclo[2.2.2]octan-Bromid
(3R)-3-[(2S)-2-Cyclopentyl-2-hydroxy-2-thien-2-ylacetoxy]-1-[3-(4-hydroxypiperidin-1-yl)propyl]-1-azoniabicyclo[2.2.2]octan-Bromid
(3R)-3-[(2S)-2-Cyclopentyl-2-hydroxy-2-thien-2-ylacetoxy]-1-[4-(4-hydroxypiperidin-1-yl)butyl]-1-azoniabicyclo[2.2.2]octan-Bromid
(3R)-3-(9,10-Dihydroanthracen-9-carbonyloxy)-1-(2-hydroxyethyl)-1-azoniabicyclo[2.2.2]octan-Bromid
(3R)-1-(2-Hydroxyethyl)-3-(9H-xanthen-9-carbonyloxy)-1-azoniabicyclo[2.2.2]octan-Bromid
(3R)-1-(2-Hydroxyethyl)-3-(9-hydroxy-9H-xanthen-9-carbonyloxy)-1-azoniabicyclo[2.2.2]octan-Bromid
(3R)-1-(2-Hydroxyethyl)-3-(9-hydroxy-9H-fluoren-9-carbonyloxy)-1-azoniabicyclo[2.2.2]octan-Bromid
(3R)-1-(2-Hydroxyethyl)-3-(9-methyl-9H-fluoren-9-carbonyloxy)-1-azoniabicyclo[2.2.2]octan-Bromid
ist.

18. Verfahren zur Herstellung von Verbindungen von Formel (I): 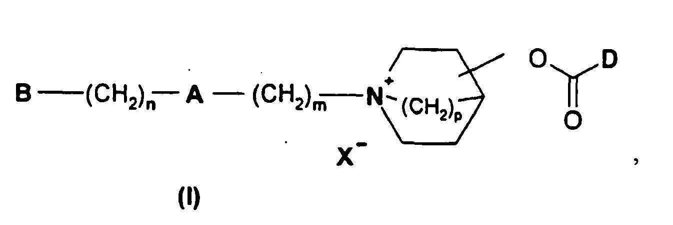 worin B, n, A, m, p und D sind, wie in einem der Ansprüche 1 bis 17 definiert, wobei das Verfahren das Quaternisieren des Stickstoffatoms des Azabicycloringes einer Verbindung von Formel (III): 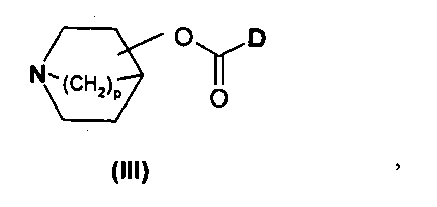 worin p und D sind, wie oben definiert, mit einem Alkylierungsmittel von Formel (II): 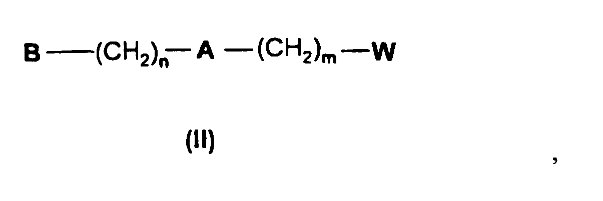 worin B, n, A und m sind, wie oben definiert, und W für eine Abgangsgruppe steht, umfasst.

19. Pharmazeutische Zusammensetzung, die eine Verbindung nach einem der Ansprüche 1 bis 17 in Vermischung mit einem pharmazeutisch verträglichen Trägerstoff oder Verdünnungsmittel umfasst.

20. Verbindung nach einem der Ansprüche 1 bis 17 oder pharmazeutische Zusammensetzung nach Anspruch 19 zur therapeutischen Verwendung in einem Verfahren zur Behandlung des menschlichen oder tierischen Körpers.

21. Verwendung einer Verbindung nach einem der Ansprüche 1 bis 17 oder einer pharmazeutischen Zusammensetzung nach Anspruch 19 bei der Herstellung eines Arzneimittels zur Behandlung einer Atemwegs-, urologischen oder gastrointestinalen Erkrankung oder Störung.

22. Verwendung nach Anspruch 21, wobei die Erkrankung oder Störung ausgewählt ist aus der Gruppe, die aus chronischer obstruktiver Lungenerkrankung (COPD), Bronchitis, bronchialer Überreaktivität, Asthma, Husten, Rhinitis, Harninkontinenz, Pollakiurie, neurogener oder instabiler Blase, Cytospasmus, chronischer Cystitis, Reizdarmsyndrom, spastischer Colitis, Diverticulitis und peptischer Ulceration besteht.

23. Kombinationsprodukt, das
(i) eine Verbindung nach einem der Ansprüche 1 bis 17; und
(ii) eine weitere Verbindung, die bei der Behandlung einer Atemwegs-, urologischen oder gastrointestinalen Erkrankung oder Störung wirksam ist,
für gleichzeitige, getrennte oder aufeinanderfolgende Verwendung umfasst.

24. Kombinationsprodukt nach Anspruch 23, das
(i) eine Verbindung nach einem der Ansprüche 1 bis 17; und
(ii) ein(en) β₂-Agonisten, Steroid, Antiallergikum, Phosphodiesterase-IV-Inhibitor und/oder Leukotrien-D4(LTD4)-Antagonisten
zur gleichzeitigen, getrennten oder aufeinanderfolgenden Verwendung bei der Behandlung einer Atemwegserkrankung umfasst.

## Revendications

1. Composé de formule (I) : 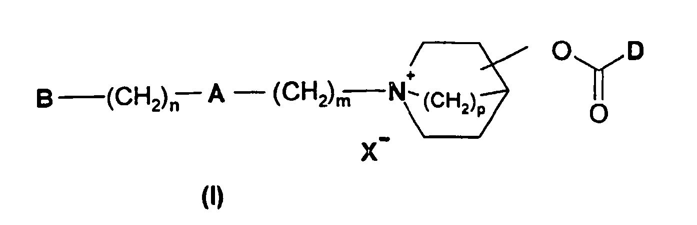 où B représente un atome d'hydrogène ou un groupe choisi parmi -R¹, -OR¹, un hydroxy, -O(CO)R¹, un cyano et un hétérocyclyle non aromatique éventuellement substitué contenant un ou plusieurs hétéroatome(s), où
R¹ est choisi parmi le groupe constitué par des atomes d'hydrogènes, un alkyle en C₁-C₈ éventuellement substitué, un alcényle en C₂-C₈ éventuellement substitué, et un cycloalkyle en C₃-C₈ éventuellement substitué
n est un nombre entier de 0 à 4 ;
A représente un groupe choisi parmi -CH₂-, -CH=CR³-, -CR³=CH-,-CR³R⁴-,-O-,-CO-,-O-(CH₂)₂-O- où R³ et R⁴ représentent chacun indépendamment un atome d'hydrogène ou un groupe alkyle en C₁-C₈ ;
m est un nombre entier de 0 à 8 ;
p est un nombre entier de 0 à 2 ;
et la substitution dans le bicycle azonia peut être à la position 2, 3 ou 4 comprenant toutes les configurations possibles des centres asymétriques ;
D est un groupe choisi parmi : 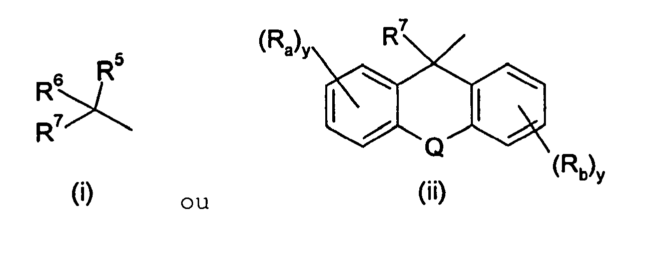 où R⁵ représente un groupe choisi parmi un phényle, un 2-thiényle, un 3-thiényle, un 2-furanyle, un 3-furanyle, lequel groupe peut être éventuellement substitué par un ou plusieurs substituants Rₐ ;
R⁶ représente un groupe choisi parmi un 2-thiényle, un 3-thiényle, un 2-furanyle, un 3-furanyle, un cycloalkyle en C₃-C₈, un cycloalcényle en C₃-C₈, un alkyle en C₁-C₈, un alcényle en C₂-C₈, un alcynyle en C₂-C₈, un benzyle et un phényléthyle, lequel groupe peut être éventuellement substitué par un ou plusieurs substituants R_{b} ;
R⁷ représente un atome d'hydrogène ou un groupe choisi parmi un hydroxyle, un hydroxyméthyle et un méthyle ;
Q représente une liaison simple ou un groupe choisi parmi -CH₂-, -CH₂CH₂-, -O-, -O-CH₂-, -S-, -S-CH₂-, et -CH=CH- ;
Rₐ et R_{b} représentent indépendamment un groupe choisi parmi les atomes d'halogène, un groupe alkyle en C₁-C₈ éventuellement substitué, un alcoxy en C₁-C₈ éventuellement substitué, un hydroxy, un trifluorométhyle, un nitro, un cyano, -COOR⁸, NR⁸R⁹ où R⁸ et R⁹ représentent indépendamment un atome d'hydrogène ou un groupe alkyle en C₁-C₈.
y est un nombre entier de 0 à 3.
X⁻ représente un anion acceptable sur le plan pharmaceutique d'un acide mono- ou polyvalent ;
à condition que le groupe B-(CH₂)ₙ-A-(CH₂)ₘ- ne soit pas un groupe alkyle en C₁-C₄ linéaire
et à condition, en outre, que le composé ne soit pas un composé parmi :
1-allyloxycarbonylméthyl-3-(2-hydroxy-2,2-di-thiophèn-2-yl-acétoxy)-1-azonia-bicyclo-[2.2.2]octane ; et
1-carboxyméthyl-3-(2-hydroxy-2,2-di-thiophèn-2-yl-acétoxy)-1-azonia-bicyclo-[2.2.2]octane.

2. Composé selon la revendication 1, où B est choisi parmi le groupe constitué par des atomes d'hydrogène, des groupes hydroxy, un alkyle en C₁-C₈ éventuellement substitué, un alcényle en C₂-C₈ éventuellement substitué, des groupes cycloalkyles en C₃-C₈ éventuellement substitués, et des groupes hétérocyclyles non aromatiques substitués au moins avec un groupe hydroxy.

3. Composé selon la revendication 1, où le groupe azoniabicyclo est substitué, sur l'atome d'azote, avec un groupe choisi parmi un allyle, un 4-méthylpent-3-ényle, un isopropyle, un cyclopropylméthyle, un isobutyle, un heptyle, un cyclohexylméthyle, un 3-cyclohexylpropyle, 3,7-diméthylocta-(E)-2,6-diényle, un 2-hydroxyéthyle, un 3-hydroxypropyle, un 4-hydroxybutyle, un 5-hydroxypentyle, un 6-hydroxyhexyle, un 2-éthoxyéthyle, un 2-(2-hydroxyéthoxy) éthyle, un 2-(2-méthoxyéthoxy)éthyle, un oxyranylméthyle, un 2-[1,3]dioxolan-2-yléthyle, un 2-[2-(2-hydroxyéthoxy)-éthoxy]éthyle, un 3-[1,3]dioxolan-2-ylpropyle, un 2-éthoxycarbonyléthyle, un 3-éthoxycarbonylpropyle, un 4-éthoxycarbonylbutyle, un 4-acétoxybutyle, un 2-cyanoéthyle, un 3-cyanopropyle, un 4-cyanobutyle, un 6-cyanohexyle, un 4,4,4-trifluorobutyle, un 3-(4-hydroxypipéridin-1-yl)propyle et un 4-(4-hydroxypipéridin-1-yl)butyle.

4. Composé selon la revendication 3, dans lequel le groupe azoniabicyclo est substitué, sur l'atome d'azote, avec un groupe choisi parmi un allyle, un 4-méthylpent-3-ényle, un isopropyle, un cyclopropylméthyle, un isobutyle, un heptyle, un 2-hydroxyéthyle, un 3-hydroxypropyle, un 4-hydroxybutyle, un 5-hydroxypentyle, un 6-hydroxyhexyle, un 2-(2-méthoxyéthoxy)éthyle, un 2-(2-hydroxyéthoxy)éthyle, un 4-éthoxycarbonylbutyle, un 4-acétoxybutyle, un 3-cyanopropyle et un 4-cyanobutyle.

5. Composé selon l'une quelconque des revendications précédentes, dans lequel p est 2.

6. Composé selon l'une quelconque des revendications précédentes, dans lequel la substitution dans le bicycle azonia se situe à la 3^{e} position et comprend toutes les configurations possibles du carbone asymétrique.

7. Composé selon la revendication 6, dans lequel l'atome de carbone à la 3^{e} position du groupe azoniabiclyclique a une configuration en R.

8. Composé selon la revendication 6, dans lequel l'atome de carbone à la 3^{e} position du groupe azoniabiclyclique a une configuration en S.

9. Composé selon l'une quelconque des revendications précédentes, dans lequel D représente un groupe de formule (i) et R⁵ représente un groupe phényle, 2-thiényle, 3-thiényle, 2-furyle ou 3-furyle non substitué.

10. Composé selon l'une quelconque des revendications précédentes, dans lequel D représente un groupe de formule (i) et R⁶ représente un groupe 2-thiényle, 3-thiényle, 2-furyle, 3-furyle ou cyclopentyle.

11. Composé selon l'une quelconque des revendications précédentes, dans lequel D représente un groupe de formule (i) et dans lequel le groupe -O-CO-C(R⁵) (R⁶) (R⁷) représente un groupe choisi parmi un 2,2-dithièn-2-ylacétoxy, un 2,2-dithièn-2-ylpropionyloxy, un 2-hydroxy-2,2-dithièn-2-ylacétoxy, un 2-hydroxy-2-phényl-2-thièn-2-ylacétoxy, un 2-fur-2-yl-2-hydroxy-2-phénylacétoxy, un 2-fur-2-yl-2-hydroxy-2-thièn-2-ylacétoxy, un (2*)-2-hydroxy-2,3-diphénylpropionyloxy, un 2-hydroxy-2-thièn-2-ylpent-4-énoyloxy, un (2S)-2-cyclopentyl-2-hydroxy-2-thièn-2-ylacétoxy et un (2R)-2-cyclopentyl-2-hydroxy-2-thièn-2-ylacétoxy.

12. Composé selon la revendication 11, dans lequel le groupe -O-CO-C (R⁵) (R⁶) (R⁷) représente un groupe choisi parmi un 2-cyclopentyl-2-hydroxy-2-thièn-2-ylacétoxy, un 2,2-dithièn-2-ylacétoxy, 2-hydroxy-2,2-dithièn-2-ylacétoxy, un 2,2-dithièn-2-ylpropionyloxy, un 2-hydroxy-2-phényl-2-thièn-2-ylacétoxy, un 2-fur-2-yl-2-hydroxy-2-thièn-2-ylacétoxy et un 2-fur-2-yl-2-hydroxy-2-phénylacétoxy.

13. Composé selon l'une quelconque des revendications précédentes, dans lequel D représente un groupe de formule (ii) et dans lequel le groupe -D-COO- représente un groupe choisi parmi un 9-méthyl-9H-fluorène-9-carbonyloxy, un 9-hydroxy-9H-fluorène-9-carbonyloxy, un 9H-xanthène-9-carbonyloxy, un 9-méthyl-9H-xanthène-9-carbonyloxy, un 9-hydroxy-9H-xanthène-9-carbonyloxy, un 9,10-dihydroanthracène-9-carbonyloxy et un 10,11-dihydro-5H-dibenzo[a,d]cycloheptène-5-carbonyloxy.

14. Composé selon la revendication 13, dans lequel le groupe -D-COO- représente un groupe choisi parmi un 9-méthyl-9H-fluorène-9-carbonyloxy, un 9-hydroxy-9H-fluorène-9-carbonyloxy, un 9H-xanthène-9-carbonyloxy, un 9-méthyl-9H-xanthène-9-carbonyloxy et un 9-hydroxy-9H-xanthène-9-carbonyloxy.

15. Composé selon l'une quelconque des revendications précédentes, dans lequel le carbone substitué par R⁵, R⁶ et R⁷ a une configuration en R.

16. Composé selon l'une quelconque des revendications 1 à 14, dans lequel le carbone substitué par R⁵, R⁶ et R⁷ a une configuration en S.

17. Composé selon la revendication 1, qui est :
le bromure de (3R)-1-allyl-3-(2,2-dithièn-2-ylacétoxy)-1 azoniabicyclo[2.2.2]octane
le bromure de (3R)-3- 2,2-dithièn-2-ylacétoxy)-1-(4-méthylpent-3-ényl)-1-azoniabicyclo[2.2.2]octane
le bromure de (3R)-1-allyl-3-(2,2-dithièn-2-ylpropionyloxy)-1-azoniabicyclo [2.2.2]octane
le bromure de (3R)-1-(4-méthylpent-3-ényl)-3-(2,2-dithièn-2-ylpropionyloxy)-1-azoniabicyclo[2.2.2]octane
le trifluoroacétate de (3R)-3-(2-Hydroxy-2,2-dithièn-2-ylacétoxy)-1-isopropyl-1-azoniabicyclo[2.2.2]octane
le trifluorocétate de (3R)-1-cyclopropylméthyl-3-(2-hydroxy-2,2-dithièn-2-ylacétoxy)-1-azoniabicyclo[2.2.2]octane
le trifluoroacétate de (3R)-3-(2-Hydroxy-2,2-dithièn-2-ylacétoxy)-1-isobutyl-1-azoniabicyclo[2.2.2]octane
le bromure de (3R)-1-heptyl-3-(2-hydroxy-2,2-dithièn-2-ylacétoxy)-1-azoniabicyclo[2.2.2]octane
le trifluoroacétate de (3R)-1-cyclohexylméthyl-3-(2-hydroxy-2,2-dithièn-2-ylacétoxy)-1-azoniabicyclo[2.2.2]octane
le trifluoroacétate de (3R)-1-(3-cyclohexylpropyl)-3-(2-hydroxy-2,2-dithièn-2-ylacétoxy)-1-azoniabicyclo[2.2.2]octane
le bromure de (3R)-1-allyl-3-(2-hydroxy-2,2-dithièn-2-ylacétoxy)-1-azoniabicyclo[2.2.2]octane
le bromure de (3R)-3-(2-hydroxy-2,2-dithièn-2-ylacétoxy)-1-(4-méthylpent-3-ényl)-1-azoniabicyclo[2.2.2]octane
le trifluoroacétate de (3R)-1-(3,7-diméthylocta-(E)-2,6-diényl)-3-(2-hydroxy-2,2-dithièn-2-ylacétoxy)-1-azoniabicyclo[2.2.2]octane
le trifluoroacétate de (3R)-3-(2-hydroxy-2,2-dithièn-2-ylacétoxy)-1-(2-hydroxyéthyl)-1-azoniabicyclo[2.2.2]octane
le trifluoroacétate de (3R)-3-(2-hydroxy-2,2-dithièn-2-ylacétoxy)-1-(3-hydroxypropyl)-1-azoniabicyclo[2.2.2]octane
le trifluoroacétate de (3R)-1-(4-hydroxybutyl)-3-(2-hydroxy-2,2-dithièn-2-ylacétoxy)-1-azoniabicyclo[2.2.2]octane
le bromure de (3R)-1-(2-éthoxyéthyl)-3-(2-hydroxy-2,2-dithièn-2-ylacétoxy)-1-azoniabicyclo[2.2.2]octane
le chlorure de (3R)-3-(2-hydroxy-2,2-dithièn-2-ylacétoxy)-1-[2-(2-hydroxyéthoxy)éthyl]-1-azoniabicyclo[2.2.2]octane
le bromure de (3R)-3-(2-hydroxy-2,2-dithièn-2-ylacétoxy)-1-[2-(2-méthoxyéthoxy)éthyl]-1-azoniabicyclo[2.2.2]octane
le bromure de (3R)-3-(2-hydroxy-2,2-dithièn-2-ylacétoxy)-1-oxyranylméthyl-1-azoniabicyclo[2.2.2]octane
le trifluoroacétate de (3R)-1-(2-[1,3]dioxolan-2-yléthyl)-3-(2-hydroxy-2,2-dithièn-2-ylacétoxy)-1-azoniabicyclo[2.2.2]octane
le trifluoroacétate de (3R)-3-(2-hydroxy-2,2-dithièn-2-ylacétoxy)-1-{2-[2-(2-hydroxyéthoxy)-éthoxy]éthyl}-1-azoniabicyclo[2.2.2]octane
le trifluoroacétate de (3R)-1-(3-[1,3]dioxolan-2-ylpropyl)-3-(2-hydroxy-2,2-dithièn-2-ylacétoxy)-1-azoniabicyclo[2.2.2]octane
le trifluoroacétate de (3R)-1-(3-éthoxycarbonylpropyl)-3-(2-hydroxy-2,2-dithièn-2-ylacétoxy)-1-azoniabicyclo[2.2.2]octane
le trifluoroacétate de (3R)-1-(4-éthoxycarbonylbutyl)-3-(2-hydroxy-2,2-dithièn-2-ylacétoxy)-1-azoniabicyclo[2.2.2]octane
le trifluoroacétate de (3R)-1-(4-acétoxybutyl)-3-(2-hydroxy-2,2-dithièn-2-ylacétoxy)-1-azoniabicyclo[2.2.2]octane
le trifluoroacétate de (3R)-1-(3-cyanopropyl)-3-(2-hydroxy-2,2-dithièn-2-ylacétoxy)-1-azoniabicyclo[2.2.2]octane
le trifluoroacétate de (3R)-1-(4-cyanobutyl)-3-(2-hydroxy-2,2-dithièn-2-ylacétoxy)-1-azoniabicyclo[2.2.2]octane
le trifluoroacétate de (3R)-1-(6-cyanohexyl)-3-(2-hydroxy-2,2-dithièn-2-ylacétoxy)-1-azoniabicyclo[2.2.2]octane
le trifluoroacétate de (3R)-3-(2-hydroxy-2,2-dithièn-2-ylacétoxy)-1-(4,4,4-trifluorobutyl)-1-azoniabicyclo[2.2.2]octane
le bromure de (3R)-1-allyl-3-(2-hydroxy-2-phényl-2-thièn-2-ylacétoxy)-1-azoniabicyclo[2.2.2]octane
le bromure de (3R)-1-allyl-3-(2-fur-2-yl-2-hydroxy-2-phénylacétoxy)-1-azoniabicyclo[2.2.2]octane
le bromure de (3R)-1-allyl-3-(2-fur-2-yl-2-hydroxy-2-thièn-2-ylacétoxy)-1-azoniabicyclo[2.2.2]octane
le bromure de (3R)-1-allyl-3-(9-méthyl-9H-fluorène-9-carbonyloxy)-1-azoniabicyclo[2.2.2]octane
le bromure de (3R)-1-allyl-3-(9-hydroxy-9H-fluorène-9-carbonyloxy)-1-azoniabicyclo[2.2.2]octane
le bromure de (3R)-3-(9-hydroxy-9H-fluorène-9-carbonyloxy)-1-(4-méthylpent-3-ényl)-1-azoniabicyclo[2.2.2]octane
le bromure de (3R)-1-heptyl-3-(9-hydroxy-9H-fluorène-9-carbonyloxy)-1-azoniabicyclo[2.2.2]octane
le bromure de (3R)-3-(9-hydroxy-9H-fluorène-9-carbonyloxy)-1-oxyranylméthyl-1-azoniabicyclo[2.2.2]octane
le bromure de (3R)-3-(9-hydroxy-9H-fluorène-9-carbonyloxy)-1-[2-(2-méthoxyéthoxy)éthyl]-1-azoniabicyclo[2.2.2]octane
le bromure de (3R)-1-(2-[1,3]dioxolan-2-yléthyl)-3-(9-hydroxy-9H-fluorène-9-carbonyloxy)-1-azoniabicyclO[2.2.2]octane
le bromure de (3R)-1-allyl-3-(9H-xanthène-9-carbonyloxy)-1-azoniabicyclo[2.2.2]octane
le bromure de (3R)-1-(4-méthylpent-3-ényl)-3-(9H-xanthène-9-carbonyloxy)-1-azoniabicyclo[2.2.2]octane
le bromure de (3R)-1-allyl-3-(9-méthyl-9H-xanthène-9-carbonyloxy)-1-azoniabicyclo[2.2.2]octane
le bromure de (3R)-1-allyl-3-(9-hydroxy-9H-xanthène-9-carbonyloxy)-1-azoniabicyclo[2.2.2]octane
le bromure de (3R)-1-(3-hydroxypropyl)-3-(9-hydroxy-9H-xanthène-9-carbonyloxy)-1-azoniabicyclo[2.2.2]octane
le bromure de (3R)-1-allyl-3-(10,11-dihydro-5H-dibenzo [a,d]cycloheptène-5-carbonyloxy)-1-azoniabicyclo[2.2.2]octane
le bromure de (3R)-1-(4-méthylpent-3-ényl)-3-(10,11-dihydro-5H-dibenzo[a,d]cycloheptene-5-carbonyloxy)-1-azoniabicyclo[2.2.2]octane
le bromure de (3R)-1-allyl-3-[(2*)-2-hydroxy-2,3-diphénylpropionyloxy]-1-azoniabicyclo[2.2.2]octane
le bromure de (3R)-3-[(2*)-2-Hydroxy-2,3-diphénylpropionyloxy)]-1-(4-méthylpent-3-ényl)-1-azoniabicyclo[2.2. 2]octane
le bromure de (3R)-1-allyl-3-(2-hydroxy-2-thièn-2-ylpent-4-énoyloxy)-1-azoniabicyclo[2.2.2]octane
le bromure de (3R)-1-(4-méthylpent-3-ényl)-3-(2-Hydroxy-2-thièn-2-ylpent-4-énoyloxy)-1- azoniabicyclo[2.2.2]octane
le bromure de (3R)-1-allyl-3-[(2S)-2-cyclopentyl-2-hydroxy-2-thièn-2-ylacétoxy]-1-azoniabicyclo[2.2.2]octane
le bromure de (3R)-1-(4-méthylpent-3-ényl)-3-[(2S)-2-cyclopentyl-2-hydroxy-2-thièn-2-ylacétoxy)-1-azoniabicyclo[2.2.2]octane
le bromure de (3R)-1-allyl-3-[(2R)-2-cyclopentyl-2-hydroxy-2-thièn-2-ylacétoxy)-1-azoniabicyclo[2.2.2]octane
le bromure de (3R)-3-[(2S)-2-cyclopentyl-2-hydroxy-2-thièn-2-ylacétoxy]-1-(2-hydroxyéthyl)-1-azoniabicyclo[2.2.2]octane
le bromure de (3R)-3-[(2S)-2-cyclopentyl-2-hydroxy-2-thièn-2-ylacétoxy]-1-(3-hydroxypropyl)-1-azoniabicyclo[2.2.2]octane
le bromure de (3R)-3-[(2S)-2-cyclopentyl-2-hydroxy-2-thièn-2-ylacétoxy]-1-(4-hydroxybutyl)-1-azoniabicyclo[2.2.2]octane
le bromure de (3R)-3-[(2S)-2-cyclopentyl-2-hydroxy-2-thièn-2-ylacétoxy]-1-[2-(2-hydroxyéthoxy)éthyl]-1-azoniabicyclo[2.2.2]octane
le bromure de (3R)-3-[(2S)-2-cyclopentyl-2-hydroxy-2-thièn-2-ylacétoxy]-1-(6-hydroxyhexyl)-1-azoniabicyclo[2.2.2]octane
le bromure de (3R)-3-[(2S)-2-cyclopentyl-2-hydroxy-2-thièn-2-ylacétoxy]-1-(5-hydroxypentyl)-1-azoniabicyclo[2.2.2]octane
le bromure de (3R)-3-[(2S)-2-cyclopentyl-2-hydroxy-2-thièn-2-ylacétoxy]-1-[3-(4-hydroxypiperidin-1-yl)propyl]-1-azoniabicyclo[2.2.2]octane
le bromure de (3R)-3-[(2S)-2-cyclopentyl-2-hydroxy-2-thièn-2-ylacétoxy]-1-[4-(4-hydroxypiperidin-1-yl)butyl]-1-azoniabicyclo[2.2.2]octane
le bromure de (3R)-3-(9,10-dihydroanthracene-9-carbonyloxy)-1-(2-hydroxyéthyl)-1-azoniabicyclo[2.2.2]octane
le bromure de (3R)-1-2-hydroxyéthyl)-3-(9H-xanthène-9-carbonyloxy)-1-azoniabicyclo[2.2.2]octane
le bromure de (3R)-1-(2-hydroxyéthyl)-3-(9-hydroxy-9H-xanthène-9-carbonyloxy)-1-azoniabicyclo[2.2.2]octane
le bromure de (3R)-1-(2-hydroxyéthyl)-3-(9-hydroxy-9H-fluorène-9-carbonyloxy)-1-azoniabicyclo[2.2.2]octane
le bromure de (3R)-1-(2-hydroxyéthyl)-3-(9-méthyl-9H-fluorène-9-carbonyloxy)-1-azoniabicyclo[2.2.2]octane.

18. Procédé de production des composés de formule (I). 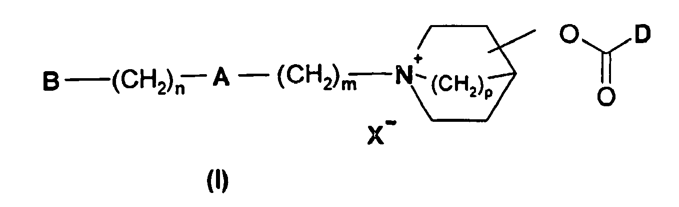 dans lequel B, n, A, m, p et D sont tels que définis dans l'une quelconque des revendications 1 à 17, lequel procédé comprend la quaternisation de l'atome d'azote de l'azabicycle d'un composé de formule (III) : 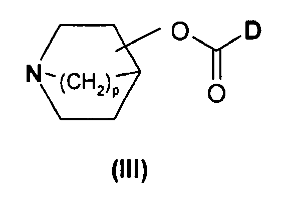 dans lequel p et D sont tels que définis ci-dessus, avec un agent d'alkylation de formule (II) : 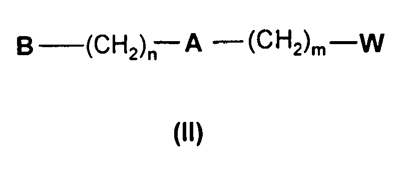 dans lequel B, n, A et m sont tels que définis ci-dessus, et W représente un groupe partant.

19. Composition pharmaceutique comprenant un composé selon l'une quelconque des revendications 1 à 17, en mélange avec un support ou diluant acceptable sur le plan pharmaceutique.

20. Composé selon l'une quelconque des revendications 1 à 17, ou composition pharmaceutique selon la revendication 19, destiné(e) à une utilisation thérapeutique dans un procédé de traitement de l'organisme humain ou animal.

21. Utilisation d'un composé selon l'une quelconque des revendications 1 à 17, ou d'une composition pharmaceutique selon la revendication 19, dans la fabrication d'un médicament pour le traitement d'une maladie ou d'un trouble respiratoire, urologique ou gastro-intestinal.

22. Utilisation selon la revendication 21, dans laquelle la maladie ou le trouble est choisi(e) parmi le groupe constitué par la bronchopneumopathie chronique obstructive (BPCO), la bronchite, l'hyperréactivité bronchique, l'asthme, la toux, la rhinite, l'incontinence urinaire, la pollakiurie, la vessie neurogène ou instable, le cytospasme, la cystite chronique, le syndrome du côlon irritable, la colite spastique, la diverticulite et l'ulcération peptique.

23. Produit de combinaison comprenant :
(i) un composé selon l'une quelconque des revendications 1 à 17 ; et
(ii) un autre composé efficace dans le traitement d'une maladie ou d'un trouble respiratoire, urologique ou gastro-intestinal.
pour une utilisation simultanée, séparée ou séquentielle.

24. Produit de combinaison selon la revendication 23 comprenant :
(i) un composé selon l'une quelconque des revendications 1 à 17 ; et
(ii) un agoniste β₂, un stéroïde, un médicament antiallergique, un inhibiteur de la phosphodiestérase IV et/ou un antagoniste du leucotriène D4 (LTD4)
pour une utilisation simultanée, séparée ou séquentielle dans le traitement d'une maladie respiratoire.
